# EUROPEAN PATENT SPECIFICATION

(11) **EP 4 117 662 B1**
(45) Date of publication and mention of the grant of the patent: **02.12.2026**
(21) Application number: 21766820.1
(22) Date of filing: 10.03.2021
(51) Int. Cl.: A61K 31/4709, A61K 31/4184, A61P 7/06, A61P 37/04, A61K 38/19

(54) **METHODS FOR TREATING NEUTROPENIA**
VERFAHREN ZUR BEHANDLUNG VON NEUTROPENIE
MÉTHODES DE TRAITEMENT DE LA NEUTROPÉNIE

(30) Priority: 10.03.2020 US 202062987707 P
(43) Date of publication of application: 18.01.2023
(73) Proprietor: X4 Pharmaceuticals, Inc., Cambridge, Massachusetts 02139 (US)
(72) Inventor: KELLEY, E. Lynne, Cambridge, Massachusetts 02139 (US); COHEN, Sarah, Cambridge, Massachusetts 02139 (US)
(74) Representative: Casalonga
(86) International application number: PCT/US2021/021713
(87) International publication number: WO 2021/183650

(56) References cited:
- WO-A1-2010/086185
- WO-A1-2020/047410
- US-A1- 2019 083 485
- US-A1- 2019 083 485
- BUSINESS WIRE: "X4 Pharmaceuticals Initiates Phase 1b Clinical Trial of Mavorixafor for the Treatment of Severe Congenital Neutropenia", BLOOMBERG PRESS RELEASE, 5 November 2019 (2019-11-05), pages 1 - 5, XP055857798, Retrieved from the Internet <URL:https://www.bloomberg.com/press-releases/2019-11-05/x4-pharmaceuticals-initiates-phase-1b-clinical-trial-of-mavorixafor-for-the-treatment-of-severe-congenital-neutropenia> [retrieved on 20211104]
- ANONYMOUS: "History of Changes for Study: NCT04154488: A Study of Mavorixafor in Participants With Severe Congenital Neutropenia and Chronic Neutropenia Disorders", CLINICALTRIALS.GOV ARCHIVE, 4 November 2019 (2019-11-04), pages 1 - 8, XP093132694, Retrieved from the Internet <URL:https://classic.clinicaltrials.gov/ct2/history/NCT04154488?V_1=View#StudyPageTop> [retrieved on 20240219]
- DALE DAVID ET AL: "Phase 2 Study of X4P-001: A Targeted Oral Therapy for Patients with WHIM Syndrome", 23RD CONGRESS OF THE EUROPEAN HEMATOLOGY ASSOCIATION (EHA), 14 June 2018 (2018-06-14), pages 1 - 1, XP093132735, Retrieved from the Internet <URL:https://www.x4pharma.com/wp-content/uploads/2022/11/EHA18_WHIM_Poster_Final.pdf> [retrieved on 20240219]
- DALE DAVID C ET AL: "Determination of Phase 3 Dose for X4P-001 in Patients with WHIM Syndrome", vol. 132, no. Supplement 1, 29 November 2018 (2018-11-29), US, pages 1102 - 1102, XP093132729, ISSN: 0006-4971, Retrieved from the Internet <URL:https://www.x4pharma.com/wp-content/uploads/2022/11/WHIM_ASH2018_Poster_Final.pdf> DOI: 10.1182/blood-2018-99-119840
- BUSINESS WIRE: "X4 Pharmaceuticals Initiates Phase 1b Clinical Trial of Mavorixafor for the Treatment of Severe Congenital Neutropenia ", BLOOMBERG PRESS RELEASE, 5 November 2019 (2019-11-05), pages 1 - 5, XP055857798, Retrieved from the Internet <URL:https://www.bloomberg.com/press-releases/2019-11-05/x4-pharmaceuticals-initiates-phase-1b-clinical-trial-of-mavorixafor-for-the-treatment-of-severe-congenital-neutropenia> [retrieved on 20211104]
- DALE DAVID C.; FIRKIN FRANK; BOLYARD AUDREY ANNA; KELLEY MERIDETH; MAKARYAN VAHAGN; GORELICK KENNETH J.; EBRAHIM TAREK; GARG VARUN: "Results of a phase 2 trial of an oral CXCR4 antagonist, mavorixafor, for treatment of WHIM syndrome", BLOOD, AMERICAN SOCIETY OF HEMATOLOGY, US, vol. 136, no. 26, 24 December 2020 (2020-12-24), US , pages 2994 - 3003, XP086813674, ISSN: 0006-4971, DOI: 10.1182/blood.2020007197

## Description

### FIELD OF THE INVENTION

The present invention relates to mavorixafor or a pharmaceutically acceptable salt or composition thereof for use in a method for treating chronic neutropenia, cyclic neutropenia, or congenital neutropenia in a patient in need thereof, wherein the patient does not have a gain-of-function mutation in the CXCR4 gene and does not have a genetic abnormality associated with WHIM syndrome, the use being defined in the claims. In another aspect, the present invention relates to mavorixafor or a pharmaceutically acceptable salt or composition thereof for use in a method for reducing the risk of an infection in a patient with neutropenia, wherein the patient with neutropenia does not have a gain-of-function mutation in the CXCR4 gene and does not have a genetic abnormality associated with WHIM syndrome, the use being defined in the claims. In another aspect, the present invention relates to mavorixafor or a pharmaceutically acceptable salt or composition thereof for use in a method for reducing the dosage of granulocyte colony-stimulating factor (G-CSF) for treating severe chronic neutropenia (SCN) in a patient in need thereof, wherein the patient does not have a gain-of-function mutation in the CXCR4 gene and does not have a genetic abnormality associated with WHIM syndrome, the use being defined in the claims..

### BACKGROUND OF THE INVENTION

Neutropenia is a condition characterized by an abnormally low concentration of neutrophils circulating in the blood, and defined by an absolute neutrophil count (ANC) below 1500 cells/µL. Severe neutropenia (ANC <500 cells/µL) is a risk factor for susceptibility to bacterial infection. Neutrophils make up the majority of circulating white blood cells and play an important role in the body's defenses against bacterial or fungal pathogenic infections and in shaping the host response to infection. In addition, neutrophils participate in immune system homeostasis. Neutropenia can be divided into congenital (i.e., present at birth) and acquired. Additionally, neutropenia can be "acute" (transient, or temporary, often as a response to specific events that deplete the body of neutrophils, such as radiation or chemotherapy), or "chronic" (a long-term or long-lasting effect that may be due to the presence of genetic abnormalities).

Acute or transient neutropenia can be caused by infectious agents, such as the typhoid-causing bacterium *Salmonella enterica;* and cytomegalovirus, as well as chemical agents, including propylthiouracil; levamisole; penicillamine; clozapine; valproic acid; and cancer chemotherapy.

Chronic neutropenia can be caused by genetic abnormalities (congenital neutropenia). Mutations in *ELANE* are the most common cause of congenital neutropenia. Other examples of genes that can be responsible for genetic causes of neutropenia include *HAX1, G6PC3, WAS, SBDS,* and others. In addition, some enzyme deficiencies can be associated with neutropenia such as glycogen storage disease 1b. Other causes of neutropenia include mitochondrial diseases, such as Pearson syndrome. Some autoimmune diseases, such as systemic lupus erythematosus ("SLE" or "lupus") may be associated with neutropenia. Aplastic anemia, due to bone marrow failure, is associated with thrombocytopenia, anemia and neutropenia; Evans syndrome is characterized by autoimmune hemolytic anemia (AIHA) and immune thrombocytopenia (ITP) and/or immune neutropenia; and Felty's syndrome is characterized by rheumatoid arthritis, splenomegaly and neutropenia. Chronic neutropenia may also be the result of nutritional deficiencies, such as abnormally low levels of copper or Vitamin B12; or chronic infections, such as with human immunodeficiency virus (HIV), the agent that causes AID syndrome.

Neutropenia may be asymptomatic and often is only diagnosed fortuitously. Today, the standard treatment for severe neutropenia is administration of granulocyte colony-stimulating factor (G-CSF). Historically, neutropenia has been treated in a host of manners, including splenectomy, corticosteroids, androgens, and immunosuppressive and immune-modulating therapies. Currently, however, these treatments are generally not recommended except in cases where treatment with G-CSF is not effective. Dale et al. (2017) Curr. Opin. Hematol. 24:46-53; Sicre de Fontbrune et al. (2015) Blood 126:1643-1650. Other treatments for neutropenia can include bone marrow transportation and/or treatment with cord blood stem cells.

WO 2020/047410 A1 discloses compositions and methods of use for treating, preventing, or ameliorating a disease, disorder, or condition associated with a chemokine receptor such as CXCR4.

"History of Changes in for Study: NCT04154488: A Study of Mavorixafor Participants With Severe Congenital Neutropenia and Chronic Neutropenia Disorders", Clinical Trials.gov archive, 4 November 2019 (2019-11-04), pages 1-8, discloses a Phase 1b clinical trial protocol regarding a study of mavorixafor in participants with severe congenital neutropenia and chronic neutropenia disorders.

Thus, there remains a need for more effective treatments of neutropenia and associated diseases. The present invention addresses this need and provides other related advantages.

### SUMMARY OF THE INVENTION

The present invention is defined by the claims. Any subject-matter falling outside the scope of the claims is provided for information only. In one aspect, the present invention relates to mavorixafor or a pharmaceutically acceptable salt or composition thereof for use in a method for treating chronic neutropenia, cyclic neutropenia, or congenital neutropenia in a patient in need thereof, wherein the patient does not have a gain-of-function mutation in the CXCR4 gene and does not have a genetic abnormality associated with WHIM syndrome, the use being defined in the claims.

In another aspect, the present invention relates to mavorixafor or a pharmaceutically acceptable salt or composition thereof for use in a method for reducing the risk of an infection in a patient with neutropenia, wherein the patient with neutropenia does not have a gain-of-function mutation in the CXCR4 gene and does not have a genetic abnormality associated with WHIM syndrome, the use being defined in the claims.

In another aspect, the present invention relates to mavorixafor or a pharmaceutically acceptable salt or composition thereof for use in a method for reducing the dosage of granulocyte colony-stimulating factor (G-CSF) for treating severe chronic neutropenia (SCN) in a patient in need thereof, wherein the patient does not have a gain-of-function mutation in the CXCR4 gene and does not have a genetic abnormality associated with WHIM syndrome, the use being defined in the claims.

Methods of treatment of the human body by therapy, referred to in this description, are not part of the present invention as such, but are described here in relation to compounds and pharmaceutical compositions for use in said methods for treatment of the human body by therapy, according to the present invention.

In some embodiments, the patient has an absolute neutrophil count (ANC) less than about 500 cells/µL.

According to the present invention, patients with neutropenia are treated with an effective amount of mavorixafor, or a pharmaceutically acceptable salt or composition thereof, in combination with other treatments for neutropenia (combination therapy). The combination therapy comprises treatment with an effective amount of granulocyte colony-stimulating factor (G-CSF).

In some embodiments, the neutropenia is chronic idiopathic neutropenia (CIN), severe chronic neutropenia (SCN), or autoimmune neutropenia (AIN). In some embodiments, the patient has a genetic abnormality selected from GSD1b, G6PC3 deficiency, GATA2 deficiency, or a genetically-defined condition with or without myeloid maturation arrest at the myelocyte/promyelocyte stage.

In some embodiments, G-CSF is co-administered to the patient at a starting dosage of about 6 mcg/kg as a twice daily subcutaneous injection (for a patient having congenital neutropenia); or about 5 mcg/kg as a single daily subcutaneous injection (for a patient having idiopathic or cyclic neutropenia). In some embodiments, the patient is already receiving G-CSF and continues chronic dosing at a dosage sufficient to maintain clinical benefits, such as daily administration in the amount of about 6 mcg/kg (for patients having congenital neutropenia); about 2.1 mcg/kg (for patients having cyclic neutropenia); or about 1.2 mcg/kg (for patients having idiopathic neutropenia).

In some embodiments, the effective amount of G-CSF is less than the approved dosage as a monotherapy for a similar patient being treated with the G-CSF.

According to the present invention, the dosage of G-CSF that is administered to the patient is reduced by at least about 25% relative to the patient's previous dose before beginning treatment with mavorixafor or a pharmaceutically acceptable salt or composition thereof. In certain embodiments, the dosage of G-CSF that is administered to the patient is reduced by at least about 50%, 75%, or 95% relative to the patient's previous dose before beginning treatment with mavorixafor or a pharmaceutically acceptable salt or composition thereof.

In certain embodiments, the dosage of G-CSF that is administered to the patient is reduced by at least about 25%, 30%, 35%, 40%, 50%, 55%, 60%, 65%, 70%, 75%, 80%, 85%, 90%, or 95%. In certain embodiments, the frequency of dosage of G-CSF is reduced, for example, reduced in frequency by at least 25%, 50%, 75%, or 90%.

In some embodiments, the present invention features a decrease in the incidence of bone pain in the patient, or across a representative group of patients. In some embodiments, the present invention features a decrease in the incidence of flu-like symptoms in the patient, or across a representative group of patients. In some embodiments, the present invention features a decrease in the incidence of a myeloid malignancy, such as myelodysplasia (MDS) or acute myeloid leukemia (AML), in the patient, or across a representative group of patients.

The patient has previously been treated with G-CSF.

In some embodiments, the patient has idiopathic neutropenia. In some embodiments, the patient has severe idiopathic neutropenia. In some embodiments, the patient has chronic neutropenia. In some embodiments, the patient has SCN, CIN, or AIN. In some embodiments, the patient has undergone genetic testing but no diagnosis of a genetic abnormality has been made. In some embodiments, the genetic testing was inconclusive. In some embodiments, the genetic testing revealed no known genetic abnormality, or a genetic abnormality not associated with neutropenia. In some embodiments, the patient has neutropenia not due to a genetic abnormality and due to one or more of an infectious, inflammatory, autoimmune, or malignant cause. In some embodiments, the malignant cause is a cancer.

In some embodiments, the patient has severe congenital neutropenia.

According to the present invention, the patient does not have a genetic abnormality associated with WHIM syndrome (a gain-of-function mutation in the *CXCR4* gene). In some embodiments, the patient has undergone genetic testing and a genetic abnormality other than one associated with WHIM syndrome has been diagnosed. WHIM-associated genetic abnormalities typically include a gain-of-function mutation in the *CXCR4* gene. In some embodiments, the patient has a congenital neutropenia. In some embodiments, the patient has a genetic abnormality selected from GSD1b, G6PC3 deficiency, GATA2 deficiency, a genetically-defined condition with myeloid maturation arrest at the myelocyte/promyelocyte stage, a genetically-defined condition without myeloid maturation arrest at the myelocyte/promyelocyte stage, or an undefined genetic abnormality.

In some embodiments, a provided method further comprises the step of obtaining a biological sample from the patient and measuring the amount of a disease-related biomarker. In some embodiments, the biological sample is a blood sample. In certain embodiments, the disease-related biomarker is selected from the group consisting of CXCR4, SDF-1α/CXCL12; and GRK3 (G protein coupled receptor kinase 3).

In certain embodiments, after commencement of administration of mavorixafor, the dosage of G-CSF administered to the patient is reduced, while maintaining absolute neutrophil counts (ANC) equal to or higher than 500 cells/µL. In certain embodiments, the dosage of G-CSF that is administered to the patient is reduced by about 25%, 30%, 35%, 40%, 50%, 55%, 60%, 65%, 70%, 75%, 80%, 85%, 90%, or 95%.

In some embodiments, the daily dose of mavorixafor, or a pharmaceutically acceptable salt or composition thereof, is from about 100 mg to about 800 mg. In some embodiments, the daily dose is about 200 mg to about 600 mg, such as about 400 mg. In some embodiments, the daily dose is administered in divided doses twice per day. In some embodiments, the daily dose is administered once per day. In some embodiments, the mavorixafor, or a pharmaceutically acceptable salt or composition thereof, is administered in a fasted state.

### BRIEF DESCRIPTION OF THE FIGURES

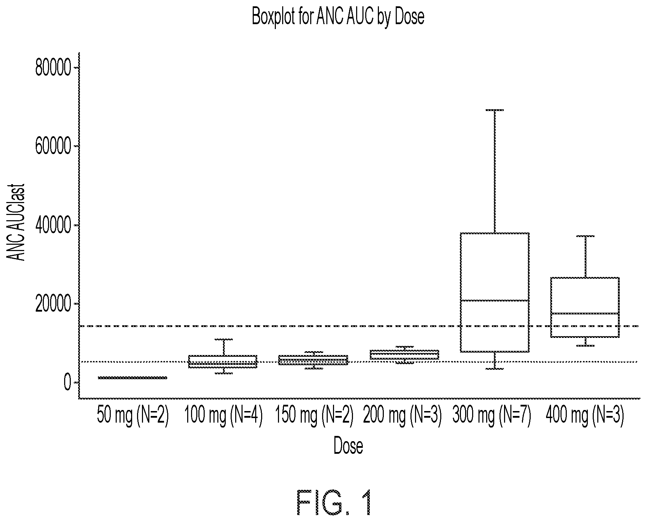

**FIG. 1** illustrates a boxplot for the area under the curve (AUC) for absolute neutrophil count (AUC_{ANC}) in patients receiving mavorixafor. In **FIG. 1****,** ALC = absolute lymphocyte count; ANC = absolute neutrophil count; AUCabs = non threshold-adjusted area under the plasma concentration curve; AUClast = area under the plasma concentration curve to the last measurable concentration. Units: cells•hr/µL. **Panel A:** ANC. **Panel B:** ALC. Symbols: bold solid line: median; cross: mean; box: 25th to 75th percentiles; whiskers: 1.5 x interquartile range; dashed line: threshold; dotted line: baseline threshold, calculated by using the geometric mean baseline ANC across all subjects, multiplied by the 24 hr dosing interval.

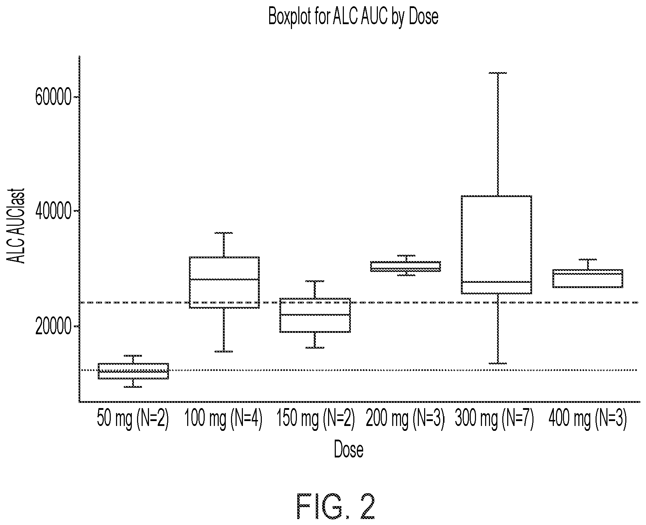

**FIG. 2** illustrates a boxplot for the area under the curve (AUC) for absolute lymphocyte count (AUC_{ALC}) in patients receiving mavorixafor. The same abbreviations are used as in **FIG. 1****.**

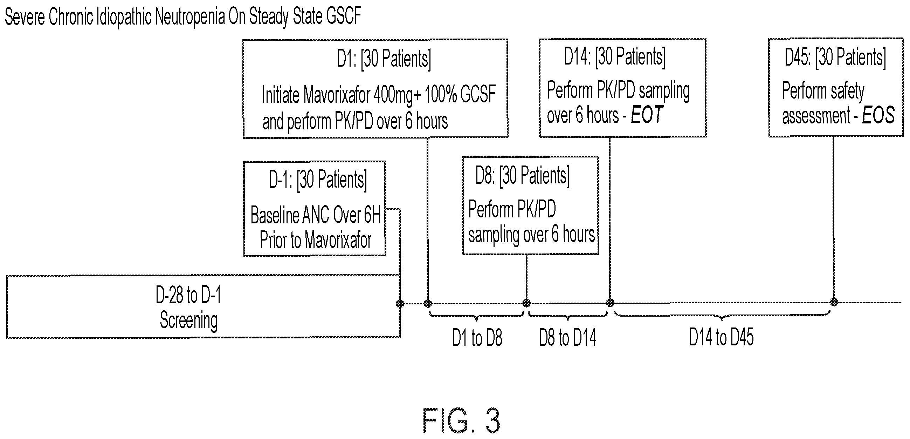

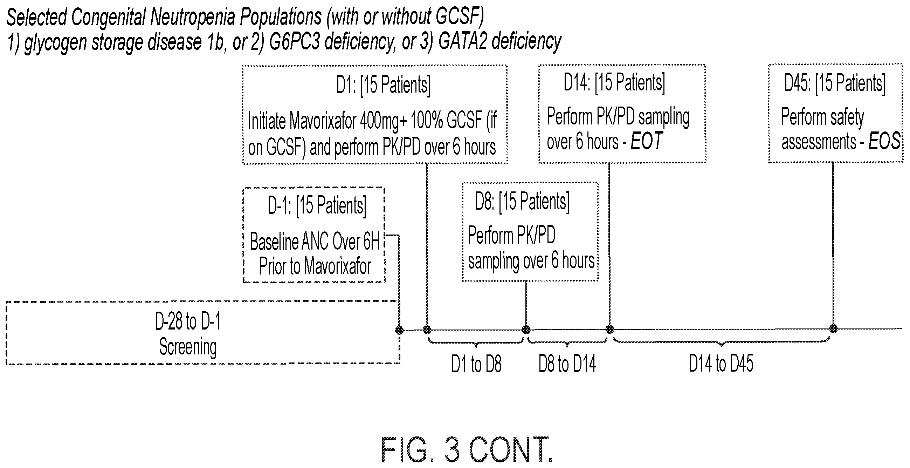

**FIG. 3** illustrates study schema for patients with severe chronic idiopathic neutropenia on steady state G-CSF; and selected congenital neutropenia patient populations (with or without G-CSF). Abbreviations: ALC = absolute lymphocyte count; ANC = absolute neutrophil count; AUC_{ALC} = area under the curve for ALC; AUC_{ANC} = area under the curve for ANC; CIN = chronic idiopathic neutropenia; D = Day; EOS = End of Study; EOT = End of Treatment; G6PC3 = glucose-6-phosphatase catalytic subunit 3; GATA2 = GATA-binding protein 2; GCSF = granulocyte-colony stimulating factor; H = hours; PD = pharmacodynamic; PK = pharmacokinetic. **Primary Endpoint:** Safety. **Secondary Endpoint:** ANC and AUC_{ANC} over 6 hours on Day 14 relative to baseline in patients with severe CIN in combination with steady state GCSF. **Exploratory Endpoints:** (1) ANC and AUC_{ANC} over 6 hours relative to baseline in patients (with or without GCSF) with glycogen storage disease 1b, G6PC3 deficiency, or GATA2 deficiency; (2) ANC and AUC_{ALC} over 6 hours relative to baseline in all patients; (3) Bone pain in patients treated with GCSF.

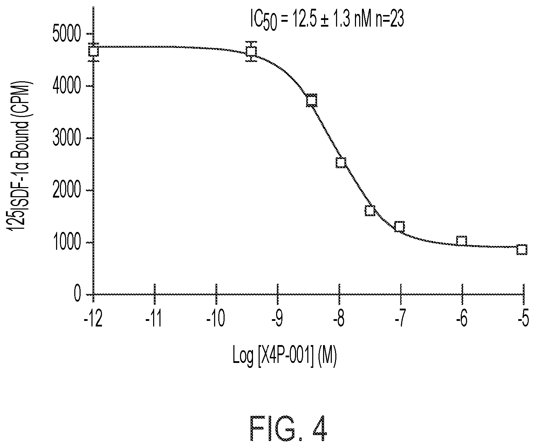

**FIG. 4** shows that mavorixafor inhibits binding of [¹²⁵I]-SDF-1α to CCRF-CEM cells (T-lymphoblastoid cell line which naturally express CXCR4 [Crump 1997]) in a heterologous competition binding assay. The data was fitted to a single site binding model and gave an IC₅₀ of 12.5 ± 1.3 nM.

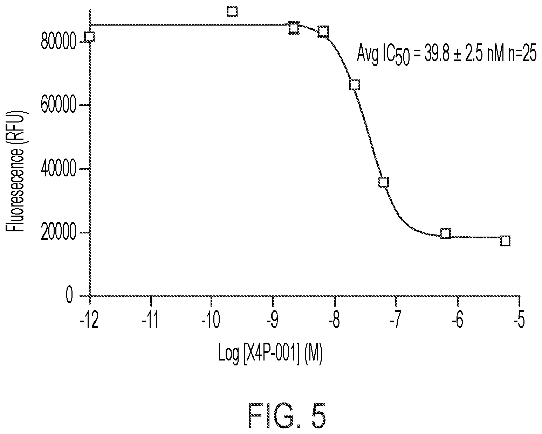

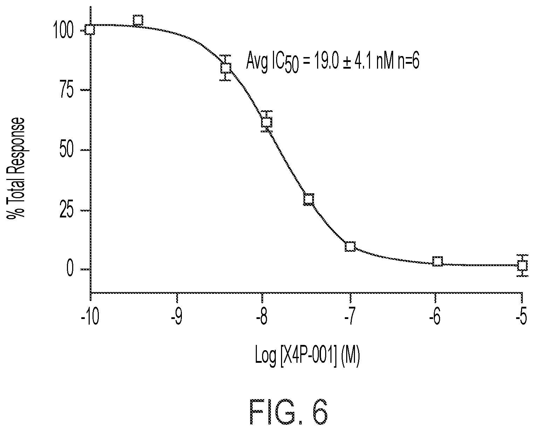

**FIG. 5** **and** **6** show that mavorixafor inhibits CXCR4 activation with IC₅₀ values of 39.8 ± 2.5 nM and 19.0 ± 4.1 nM in the Eu-GTP binding and [³⁵S]-GTPγS assays, respectively.

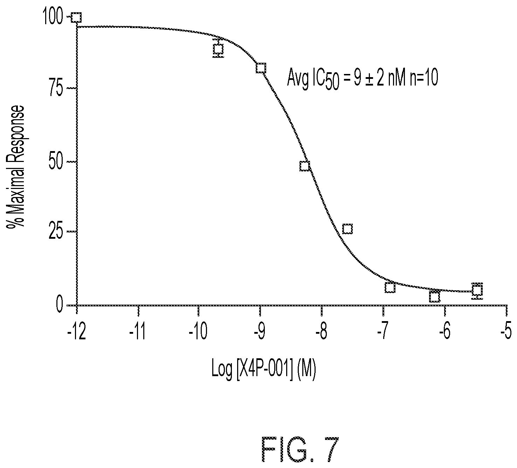

**FIG. 7** shows that, upon activation of a G-protein coupled receptor, intracellular signaling pathways are triggered resulting in the release of calcium from intracellular stores. This calcium flux can be assayed using a calcium-chelating molecule, Fluo-4, which fluoresces upon binding calcium. Mavorixafor was able to inhibit SDF-1α (2.5 nM SDF-1α) mediated calcium flux in CCRF-CEM cells with an IC₅₀ of 9.0 ± 2.0 nM.

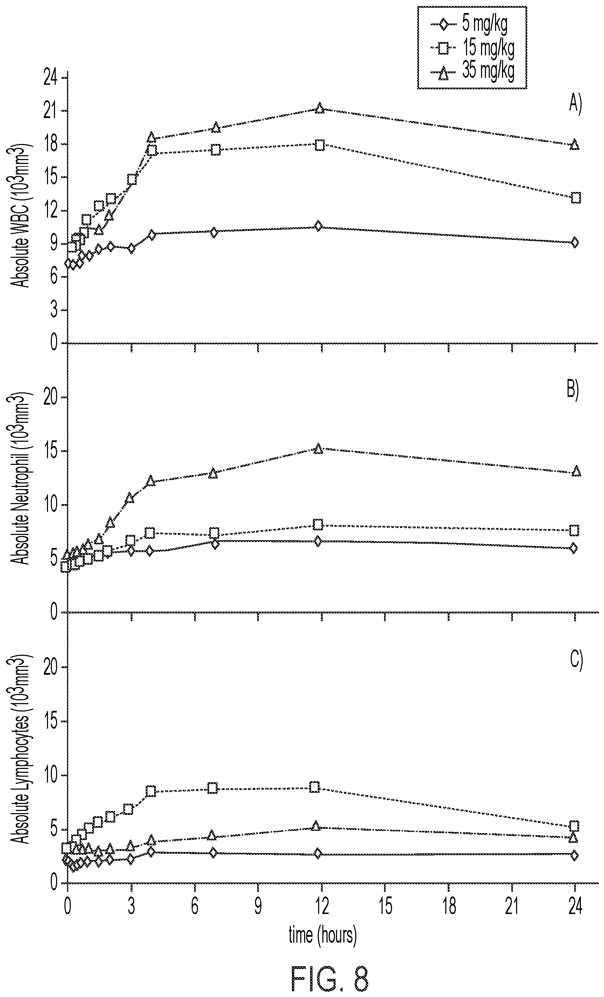

**FIG. 8** shows the effect of mavorixafor on WBC and absolute neutrophil and lymphocyte counts in male beagle dogs. Maximal increases in WBC occurred 4-12 hours post-dose. Peak elevations ranged from 1.8-2.9-fold above baseline values at the 15 and 35 mg/kg dose levels, with somewhat lower (1.5-fold) elevations observed at the 5 mg/kg dose level. Although limited by the small sample size, these results suggest that maximal increases may have been achieved at the higher dose levels. WBC, neutrophil, and lymphocyte counts remained elevated at the 15 and 35 mg/kg dose levels at 24 hours, with evidence of return to baseline. No other hematological effects were observed.

### DETAILED DESCRIPTION OF CERTAIN EMBODIMENTS OF THE INVENTION

It has now been found that CXCR4 inhibitors such as mavorixafor (X4P-001) are useful for treating neutropenia, such as severe chronic idiopathic neutropenia, including certain genetically defined congenital forms of neutropenia, in combination with a standard of care treatment such as G-CSF.

As used herein, the term "neutropenia" means that a patient has an absolute neutrophil count (ANC) that is at or below about 1000 cells per µL. As used herein, "severe neutropenia" means that the patient has an ANC that is at or below 500 cells/µL.

As used herein, the term "chronic neutropenia" is defined as neutropenia lasting for a period of at least three (3) months. The term "idiopathic" as applied herein to neutropenia means that the neutropenia is not attributable to drugs, or to a specific identified genetic, infectious, inflammatory, autoimmune or malignant cause.

As used herein, the "congenital neutropenia" condition includes patients who exhibit neutropenia (or severe neutropenia) due to a genetically defined mutation such as glycogen storage disease type 1b (GSD1b) due to mutations in *SLC37A4,* glucose-6-phosphatase catalytic subunit 3 (G6PC3) deficiency due to mutations in *G6PC3;* or GATA-binding protein 2 (GATA2) deficiency due to mutations in *GATA2.* Other genetically-defined conditions without myeloid maturation arrest at the myelocyte/promyelocyte stage are also included in this definition.

### Neutropenias Such as Chronic Idiopathic Neutropenia (CIN), Severe Chronic Neutropenia (SCN), and Autoimmune Neutropenia (AIN)

Chronic neutropenia is defined as neutropenia lasting for at least 3 months. The term "idiopathic" indicates that the neutropenia is not attributable to drugs or an identified genetic, infectious, inflammatory, autoimmune, or malignant causes. Thus, the diagnosis of chronic idiopathic neutropenia (CIN) is one made by exclusion of other causes. Finally, the neutropenia is "severe" when the absolute neutrophil count (ANC) is below 500 cells/µL. There is also overlap of patients with the diagnosis of CIN and "autoimmune neutropenia" (AIN) because it is difficult to accurately detect circulating antibodies directed toward antigens present on the surface of neutrophils, and clinical interpretation of the anti-neutrophil antibody test result is also difficult. (Dale, Current Opin Hematol, 2018). The estimated adult prevalence of severe chronic idiopathic neutropenia is approximately 5 per million (Dale and Bolyard (2017) Curr. Opin. Hematol. 24:46-53). There is a female predominance of CIN (Kyle and Linman (1968) N. Engl. J. Med. 279:1015-1019). Distinct pathophysiologic mechanisms have been found, including decreased production, enhanced peripheral removal, and excessive margination of neutrophils (Greenberg et al. (1980) Blood 55:915-921). Neutrophil counts < 500 cells/µL are associated with a higher risk of infections. In one study, the bone marrow was analyzed in approximately one third of a series of 108 patients and results were normal in 34% of patients; late maturation arrest was seen in 31% of the patients; granulocytic hypoplasia was observed in 15% of the patients; and 20% of the patients had increased cellularity (Sicre de Fontbrune 2015). A randomized, controlled trial of G-CSF for treatment of severe chronic neutropenia, including 42 patients with CIN, established G-CSF as an effective therapy for this condition (Dale (1993) Blood 81:2496-2502).

In some embodiments, treatment of particular sub-populations of patients with mavorixafor, or a pharmaceutically acceptable salt thereof, is particularly effective.

In some embodiments, the patient is male. In some embodiments, the patient is female.

In some embodiments, the patient is less than 50 years old. In some embodiments, the patient is at least 50 years old.

The patient has previously been treated with G-CSF.

In some embodiments, the mavorixafor, or a pharmaceutically acceptable salt thereof, and the G-CSF, or another granulocyte-colony stimulating factor treatment such as those described herein, act synergistically. Synergism includes, for example, more effective treatment of the disease than with either agent alone; or a lower dose of one or both agents providing effective treatment for the disease than would be the case if either agent were used alone.

In some embodiments, the patient is currently being treated with G-CSF. In some embodiments, the dose and/or frequency of administration of G-CSF (while maintaining effectiveness of the treatment regimen) is/are reduced after treatment with mavorixafor, or a pharmaceutically acceptable salt thereof, is commenced.

In some embodiments, the patient has idiopathic neutropenia. In some embodiments, the patient has severe idiopathic neutropenia. In some embodiments, the patient has chronic neutropenia. In some embodiments, the patient has SCN, CIN, or AIN. In some embodiments, the patient has undergone genetic testing but no diagnosis of a genetic abnormality has been made. In some embodiments, the genetic testing was inconclusive. In some embodiments, the genetic testing revealed no known genetic abnormality, or a genetic abnormality not associated with neutropenia. In some embodiments, the patient has neutropenia not due to a genetic abnormality and due to one or more of an infectious, inflammatory, autoimmune, or malignant cause. In some embodiments, the malignant cause is a cancer.

In some embodiments, the patient has severe congenital neutropenia.

In some embodiments, the patient has undergone genetic testing and a genetic abnormality other than one associated with WHIM syndrome has been diagnosed. In some embodiments, the patient has a congenital neutropenia. In some embodiments, the patient has a genetic abnormality selected from GSD1b, G6PC3 deficiency, GATA2 deficiency, a genetically-defined condition without myeloid maturation arrest at the myelocyte/promyelocyte stage, or an undefined genetic abnormality.

### Glycogen Storage Disease 1b

Glycogen storage disease type 1b (GSD1b) is an autosomal recessive disorder with an incidence of 2 per million (Chou and Mansfield (2003) in: Broer and Wagner, eds. Membrane Transporter Diseases. New York: Springer; 191-205). It is caused by homozygous or compound heterozygous mutations in the *SLC37A4* gene coding for the ubiquitously expressed glucose 6-phosphate (G6P) transporter (G6PT). The G6PT enzyme is a transmembrane protein providing a selective channel between the endoplasmic reticulum lumen and the cytosol. The G6PT translocates G6P from the cytoplasm into the lumen of the endoplasmic reticulum in glucose-6-phosphatase (G6Pase)-α or by a ubiquitously expressed G6Pase-β. In neutrophils and macrophages, the G6PT/G6Pase-β complex preserves energy homeostasis and functionality (Chou et al. (2010) Curr. Opin. Hematol. 17:36-42). Specifically, the enzyme is made up of 3 separate transporting subunits referred to as G6PT1 (subunit 1), G6PT2 (subunit 2), and G6PT3 (subunit 3). Subunit 1, G6PT1, transports G6P from the cytosol into the lumen of the endoplasmic reticulum where it is hydrolyzed by the catalytic subunit of G6Pase. After hydrolysis, glucose and inorganic phosphate are transported back into the cytosol by G6PT2 and G6PT3, respectively (Parker (2001) Drugs Fut. 26:687). The absence of a functional G6PT1 enzyme causes the disease GSD1b.

Because neutrophil function is linked to the regulation of glucose and G6P metabolism by the G6PT/G6Pase-β complex, most of GSD1b patients present with neutropenia, neutrophil dysfunction, and recurrent infections in the context of a broader metabolic disorder also characterized by hypoglycemia, excessive glycogen accumulation in the liver and kidney, and abnormal metabolic serum profiles. Up to 77% of neutropenic patients also develop inflammatory bowel disease (IBD).

A collaborative European study showed that 54 of a cohort of 57 GSD1b patients had neutropenia. Of these, 64% were first neutropenic before the age of 1 year, and a further 18% became neutropenic between the ages of 6 to 9 years (Visser et al. (2000) J Pediatr. 137:187-91). Neutrophils from GSD1b patients exhibit impaired mobility, chemotaxis, and calcium mobilization, as well as diminished respiratory burst and phagocytotic activities. Human GSD1b neutrophils have been found to show signs of apoptosis with increased caspase activity, condensed nuclei, and perinuclear clustering of mitochondria to which the proapoptotic BCL2 member BCL2 associated X had translocated already (Kim et al. (2008) Blood. 111: 5704-11). G-CSF added to in vitro cultures did not rescue the GSD1b neutrophils from apoptosis as occurred with G-CSF (Ueno et al. (1986) Eur J Pediatr. 145:312-14; Roe et al. (1986) J Pediatr 109:55-9). In patients, the bone marrow aspirations show hypercellularity due to myeloid hyperplasia and resulting from an arrest of myeloid maturation.

Neutropenia and/or neutrophil dysfunctions predispose GSD1b patients to frequent bacterial infections, aphthous stomatitis and inflammatory bowel disease. (Melis et al. (2014) Italian J. Pediatrics 40:30). Splenomegaly is the dose-limiting adverse event (AE) in GSD1b patients treated with G-CSF, leading to pain and early satiety. While clinical observations and records attest to reduced frequency of infectious events, fever and recurrent infections remain a significant problem despite G-CSF treatment. In one study, the majority of patients being treated with G-CSF developed myelodysplasia (MDS) or acute myeloid leukemia (AML). (Dale et al. (2019) Curr Opin Hematol. 26:16-21; Visser et al. (2000); Visser et al. (2002) Eur J Pediatr. 161(Suppl 1):S83-7). Without wishing to be bound by theory, it is believed that the development of AML in GSD1b patients may be linked to chronic G-CSF use or to the natural course of the disease or a combination of both (Chou et al. (2010) Curr Opin Hematol. 17:36-42).

### G6PC3 Deficiency

The *G6PC3* gene encodes the ubiquitously expressed G6PC3. In 2009, Boztug showed that effective function of G6PC3 underlies a severe congenital neutropenia syndrome associated with cardiac and urogenital malformations (Boztug et al. (2009) N Engl J Med. 360:32-43).

As of 2013, 57 patients with G6PC3 deficiency have been described in the literature (Banka and Newman (2013) Orphanet J Rare Dis. 8:84). There have been 91 cases reported globally with an estimated incidence of 0.4 in 1,000,000 births and primarily of Turkish, Pakistani, and French descent. G6PC3 deficiency usually presents in the first few months of life with recurrent bacterial infections and ANC counts ranging from 120 to 550 cells/µL (McDermott et al. (2010) Blood. 116:2793-802). The first serious infection can occur at any age, ranging from immediately after birth to adulthood (Banka (2015, in Gene Reviews, Adam et al, editors. University of Washington, Seattle; 1993-2019). Reported common bacterial infections are respiratory tract infections, otitis media, stomatitis, urinary tract infections, pyelonephritis, skin abscesses, cellulitis, and sepsis. G6PC3 deficiency varies in its severity and associated clinical features. It may present as non-syndromic, with isolated severe congenital neutropenia or, more frequently, syndromic, with cardiovascular and/or urogenital features. A subset of those with syndromic disease present a severe form (Dursun syndrome), due to the additional involvement of myeloid cells, characterized by primary pulmonary hypertension in the newborn period and minor dysmorphic features (Banka 2015). While it is estimated that nearly 10% of G6CP3 deficiency is the non-syndromic form, this could be an underestimate due to ascertainment bias (i.e., selection of more severe phenotypes for testing of G6PC3 in previous studies) (Banka 2013). It is also possible that some patients who initially present with the non-syndromic form may develop features of the classic form later in life (Banka 2015). While bone marrow analysis may show maturation arrest in the myeloid lineage, other G6PC3 deficiency patients may have hyper- or normo-cellular marrows (McDermott 2010; Banka et al. (2011) Am J Hematol. 86:235-7).

### GATA2 Deficiency

GATA2 deficiency is an autosomal dominant bone marrow failure disorder with systemic features caused by heterozygous germline mutation in 1 of 2 copies of the *GATA2* gene encoding the GATA2 protein. Germline *GATA2* mutations have been detected among patients presenting with severe congenital neutropenia, suspected aplastic anemia, B-cell immunodeficiency, juvenile myelodysplastic syndrome (MDS), chronic myelomonocytic leukemia, severe Epstein-Barr virus infections and Epstein-Barr-associated cancers, B-cell acute lymphoblastic leukemia, and other unexplained cases of bone marrow failure (Crispino and Horwitz (2017) Blood. 129:2103-10). In 2017 and 2018, 457 cases of GATA2 deficiency were reported globally. Patients presented with varying ANC levels of 1100 to 8460 cells/µL (Maciejewski-Duval et al. (2016) J Leukoc Bio. 99:1065-76) and often low lymphocyte levels from 112 to 1987 cells/µL (Vinh et al. (2010) Blood. 115:1519-29) or 490 to 2900×10⁶/mL (Maciejewski-Duval 2016). The bone marrow of patients with GATA2 deficiency has been reported to range from a hypocellular marrow with normal cytogenetics to hypercellular marrow with unfavorable cytogenetics to overt AML with 85% monoblasts (Hickstein (2018) Blood. 131:1272-74). The GATA 2 deficiency phenotype ranges from immunodeficiency to aplastic anemia to MDS to leukemia (Hickstein 2018).

The diagnosis is further challenging because of the observation that while germline mutations in *GATA2* are responsible for GATA2 deficiency, acquired mutations are seen in MDS, AML, and in blast crisis transformation of chronic myeloid leukemia. In fact, GATA2 deficiency is currently the most common hereditary cause of MDS in children and adolescents. The natural history of GATA2 deficiency is highly variable, even in individuals with identical mutations. Infectious complications are common in GATA2 deficiency and result from the selective cellular deficiency profile, namely deficiency of monocytes, natural killer cells, and B lymphocytes. Hematologic manifestations of GATA2 deficiency are mainly progressive cytopenias, with a possible progression from a normocellular marrow to hypocellular MDS or AML.

Approximately half of patients with GATA2 deficiency receive allogeneic hematopoietic stem cell transplant (Hickstein 2018), and allogeneic stem cell transplantation is the only curative therapy for GATA2 deficiency. There are no clear guidelines regarding the monitoring schedule or the ideal prophylaxis for asymptomatic *GATA2* patients. However, proposals include monitoring peripheral blood counts every 3 to 6 months and bone marrow biopsy with cytogenetics every 1 to 2 years and to transplant before the development of severe end organ damage or leukemia (Hsu et al. (2015) Curr. Opin. Allergy Clin. Immunol. 15:104-9).

Mavorixafor may prove a useful bridge to transplant because of the potential to improve both the neutropenia and the lymphopenia in these patients.

### Combination of G-CSF and CXCR4 Inhibition for Treatment of Chronic Neutropenia

Granulocyte colony-stimulating factor is currently the standard of care for severe chronic neutropenia (SCN). Indeed, in patients diagnosed with chronic neutropenia, particularly those with severe neutropenia with ANC < 500 cells/µL, daily (or multiple times a week) injections of G-CSF are commonly given to increase the ANC and reduce the risk of infections. The efficacy of G-CSF in this indication was proven by a placebo-controlled clinical trial that demonstrated G-CSF safety and efficacy in reducing the risk of infection in patients with SCN of various etiologies (Dale et al. (1993) Blood. 81:2496-502).

For treatment of severe, chronic neutropenia, Neupogen^{®} (filgrastim or G-CSF) is indicated at a starting dosage 6 mcg/kg as a twice daily subcutaneous injection (congenital neutropenia); or 5 mcg/kg as a single daily subcutaneous injection (idiopathic or cyclic neutropenia). It is further indicated that the starting dosage by followed by chronic daily administration in order to maintain clinical benefits. The indicated chronic daily administration is in the amount of 6 mcg/kg (congenital neutropenia); 2.1 mcg/kg (cyclic neutropenia); and 1.2 mcg/kg (idiopathic neutropenia). Neulasta^{®} (pegfilgrastim or pegylated G-CSF) is not presently approved for treatment of severe, chronic neutropenia other than in patients receiving myelosuppressive chemotherapy or radiation. It is available in a 6 mg/0.6 mL single-dose prefilled syringe, which may be administered once per chemotherapy cycle, or in two doses of 6 mg each, one week apart, for subjects who have been exposed to radiation levels in excess of 2 gray (Gy). Neulasta^{®} is also available for use with the "on-body injector" or OBI, which is co-packaged with a prefilled syringe, and which administers the Neulasta^{®} dose over a period of approximately 45 minutes, beginning approximately 27 hours after the OBI is applied to the subject's skin.

Bone pain experienced with administration of G-CSF has commonly been treated with acetaminophen and nonsteroidal anti-inflammatory agents as first line therapy, while antihistamines, such as loratidine (10 mg oral); or combinations of famotidine and loratadine; opioids; and dose reduction of G-CSFs are considered as second line therapy (Lambertini et al. (2014) Crit. Rev. Oncol. Hematol. 89:112-128).

Without wishing to be bound by theory, it is believed that G-CSF's effect on the bone marrow release of neutrophils is mediated in part by interfering with CXCL12 availability at the level of the CXCR4 receptor, with minimal effects on other hematopoietic cell types.

Granulocyte-colony stimulating factor treatment induces a decrease in CXCL12 expression in the bone marrow (Semerad et al. (2002) Immunity. 17:413-23; Levesque et al. (2003) J Clin Invest. 111:187-96), and G-CSF leads to decreased surface expression of CXCR4 on neutrophils (Kim et al. (2006) Blood. 108:812-20). In fact, G-CSF does not stimulate neutrophil release from the bone marrow in the absence of CXCR4 signals (Eash et al. (2009) Blood. 113:4711-19).

Without wishing to be bound by any particular theory, it is believed that certain patient populations having neutropenia could be treated effectively with a combination of specific CXCR4 inhibitors, such as mavorixafor, and G-CSF. It is further believed that such treatment produces a significant increase in patient baseline ANC. It is also believed that subjects with neutropenia (or severe neutropenia) who are currently treated with G-CSF, including those subjects who experience bone pain or other serious adverse effects of receiving G-CSF, could be treated with a CXCR4 inhibitor, such as mavorixafor, and that treatment with CXCR4 inhibitor allows for a reduction in the dosage and/or frequency of treatment with G-CSF, while still maintaining an ANC above a minimum threshold (e.g., ANC of at least 500/µL) to prevent infections and other manifestations of neutropenia (e.g., oral ulcers).

### Combination with Mavorixafor

In some embodiments of the present invention, mavorixafor is administered orally (PO) to the patient at a dose regimen of about 400 mg once daily (QD).

In some embodiments, the dosage of CXCR4 inhibitor is a well-tolerated dose that achieves a satisfactory therapeutic result, without causing any severe or treatment-limiting toxicities.

As used herein, the term "well-tolerated" in reference to a dose of mavorixafor or other CXCR4 inhibitor means a dose that can be given to a patient without the patient experiencing any treatment-limiting toxicities. As used herein, "treatment-limiting toxicities" (TLTs) means that the patient experiences one or more of the toxicities in **Table 1:**

**Table 1: Treatment-Limiting Toxicities**

| **Toxicity** | **Treatment-Limiting Toxicity Criteria** |
|---|---|
| **Hematology** | Grade 4 neutropenia lasting more than 7 consecutive days |
| | Grade 3 or 4 neutropenia with fever (temperature of > 38.5°C) |
| | Grade 4 thrombocytopenia, or Grade 3 thrombocytopenia with bleeding |
| | Grade 4 anemia |
| | Grade 4 lymphopenia |
| **Non-Hematology Events** | Any ≥ Grade 3 clinical events or laboratory events, except for the events described below, which are TLTs only if they meet the criteria below. |
| **Gastrointestinal** | Grade 3 or 4 nausea, vomiting, or diarrhea lasting ≥ 48 hours despite optimal medical management |
| **Hepatobiliary** | ≥ Grade 2 total bilirubin elevation with ≥ Grade 2 ALT/AST elevation |
| | ≥ Grade 3 ALT/AST elevation lasting ≥ 5 days or Grade 4 ALT/AST elevation |
| **Pneumonitis** | Grade 2 pneumonitis lasting > 7 days despite optimal treatment |
| **Hypertension** | Grade 3 hypertension lasting > 7 days despite optimal treatment |
| **Infection** | Grade 3 infection or fever in the absence of neutropenia lasting > 5 days |
| **Electrolytes** | Grade 3 electrolyte abnormalities lasting > 7 days |
| **Rash and/or photosensitivity** | ≥ Grade 3 rash or photosensitivity lasting > 7 days despite optimal treatment |
| **Fatigue** | Grade 3 electrolyte abnormalities lasting > 7 days |
| **Immune-related toxicities (except pneumonitis)** | Grade 3 immune related toxicities lasting > 7 days despite optimal treatment |
| **Others** | Any other ≥ Grade 2 toxicity that, in the opinion of a treating physician is considered to be a clinically unacceptable risk |

| | |
|---|---|
| **Grading:** As defined by the National Cancer Institute [NCI] Common Terminology Criteria for Adverse Events, version 4.03). **Abbreviations:** ALT = alanine aminotransferase; AST = aspartate aminotransferase; TLT = treatment-limiting toxicity. | |

Effective targeted treatments for neutropenia, like mavorixafor, are needed for the management of patients because, for example, of the significant side effects associated with G-CSF. Mavorixafor can be administered orally (PO) once daily (QD), which in addition to being a targeted treatment, makes it an excellent candidate in a chronic treatment setting that would be required for patients with SCN or CIN. In some embodiments, mavorixafor is administered orally (PO) once daily (QD). In some embodiments, mavorixafor is administered orally (PO) twice daily (BD).

### Treatment of Neutropenia Such As SCN, CIN, and AIN with Mavorixafor and Treatment Duration

Under basal conditions, most neutrophils reside in the bone marrow, and this pool of neutrophils can be mobilized into the blood physiologically in response to infection or stress, or upon CXCR4 antagonist administration, providing a mechanism to rapidly increase neutrophil delivery to sites of infection. Humans and mice treated with a selective CXCR4 antagonist rapidly mobilize neutrophils into the blood (Liles 2003; Suratt 2004; Broxmeyer et al. (2005) J. Exp. Med. 201:1307-1318). Transgenic mice carrying a myeloid-specific deletion of CXCR4 display marked neutrophilia, thus confirming the key role of CXCR4 signaling in the regulation of neutrophil homeostasis. CXCR4 maintains neutrophil homeostasis primarily by regulating neutrophil release from the bone marrow (Eash 2009).

It is anticipated that most patients seeking treatment for chronic neutropenia and severe CIN will currently be receiving treatment with G-CSF, because this is the present standard of care (Dale, Blood 1993). In some embodiments, the patient has CIN.

While neutrophils have been reported to have a very short half-life of 8 to 16 hours under basal conditions (Lord 1991; Dresch 1975), new information has shown that under homeostatic conditions, the average circulatory neutrophil lifespan is 5.4 days (Pillay 2010). Today it is estimated that mature neutrophils have a typical circulating half-life of 6-8 h in the blood and then migrate through tissues for ~2-3 days. Their relatively short lifespan is devoted largely to surveillance for invading microorganisms. During infection, the neutrophil lifespan is extended, granulopoiesis increases, and large numbers of neutrophils are rapidly recruited to the site(s) of infection (*Neutrophil Methods and Protocols,* Third Edition, Mark T. Quinn, Humana Press, 2020). CXCL12/CXCR4 signaling plays a key role in controlling neutrophil homeostasis (Link 2005) and CXCR4 is a key regulator of neutrophil release from the bone marrow under basal and stress granulopoiesis conditions (Eash et al., Blood 2009). The generation of a mature neutrophil from the myeloblast stage takes approximately 14 days. Bainton et al., (1971) J. Exp. Med. 134:907-34.

### Treating Neutropenia and Related Disorders

Effective targeted treatments for neutropenia, like mavorixafor, are needed for the management of patients. Mavorixafor can be administered orally (PO) and once-daily (QD), which in addition to being a targeted treatment, makes it an excellent candidate in a chronic treatment setting that would be required for patients with SCN or CIN.

The cognate ligand for the CXCR4 receptor is stromal cell-derived factor 1-alpha (SDF-1α), also known as C-X-C motif chemokine 12 (CXCL12), which is involved with numerous physiologic processes and plays a central role in hematopoietic cell homing to, and release from, the bone marrow (Lapidot 2002). In patients with WHIM syndrome, gain-of-function mutations in the *CXCR4* gene prevent the normal release of mature neutrophils from the bone marrow into the blood (Kawai 2005).

CXCR4 is a G protein-coupled receptor and engagement by SDF-1α induces typical activation of G protein-dependent pathways of a chemokine receptor (Baggiolini 1998, Zlotnik 2000). These processes are regulated in a timely manner by the recruitment of β-arrestin to the receptor that precludes further G-protein activation (i.e., desensitization) and leads to receptor internalization.

Mavorixafor is a small molecule antagonist of CXCR4 having the potential to block the enhanced signaling activity of wild-type and mutant CXCR4 receptors, resulting in an increase in the number of circulating white blood cells.

In some embodiments, mavorixafor or a pharmaceutically acceptable salt thereof is dosed by oral administration of up to 400 mg daily. In some embodiments, the dose is selected to provide consistent clinically relevant elevations of both ANC and ALC, with low risk of significant adverse effects. Dosage of 400 mg per day BID for 3.5 days (healthy volunteers) (Stone 2007) and 200 mg BID for 8-10 days (healthy volunteers and HIV patients) were well-tolerated with no pattern of adverse events or clinically significant laboratory changes. These studies also demonstrated pharmacodynamics activity, with dose- and concentration-related changes in circulating white blood cells (WBCs); and a high volume of distribution (VL), suggesting high tissue penetrance.

The inventors conceived that CXCR4 antagonism by mavorixafor may provide significant treatment benefits in patients with neutropenia, particularly for patients with chronic neutropenia, including congenital neutropenia and severe congenital neutropenia, and severe chronic idiopathic neutropenia (CIN) as described in the present application.

Administration of mavorixafor inhibits SDF-1α (CXCL12) binding to CXCR4 and CXCR4+CEM-CCRF cells. Administration of mavorixafor also inhibits CXCR4 cell signaling and SDF-1α induced calcium flux. In this manner, X4P-001 inhibits SDF-1α stimulated CCRF-CEM chemotaxis.

Moreover, the inventors conceived that such a result might be achieved with comparatively little toxicity since CXCR4-targeted drugs are specifically targeted and do not induce cell cycle arrest in normal proliferating cell populations. Accordingly, the present invention provides significant advantages in treatment outcomes utilizing the effects and the low toxicity of the CXCR4 inhibitor mavorixafor (also known as X4P-001; AMD070; or AMD11070).

Thus, in one aspect, the present invention provides mavorixafor or a pharmaceutically acceptable salt or composition thereof for use in a method for treating chronic neutropenia, cyclic neutropenia, or congenital neutropenia in a patient in need thereof, wherein the patient does not have a gain-of-function mutation in the CXCR4 gene and does not have a genetic abnormality associated with WHIM syndrome, said use being defined in the claims.

In another aspect, the present invention provides Mavorixafor or a pharmaceutically acceptable salt or composition thereof for use in a method for reducing the risk of an infection in a patient with neutropenia, wherein the patient with neutropenia does not have a gain-of-function mutation in the CXCR4 gene and does not have a genetic abnormality associated with WHIM syndrome, said use being defined in the claims.

In another aspect, the present invention provides Mavorixafor or a pharmaceutically acceptable salt or composition thereof for use in a method for reducing the dosage of granulocyte colony-stimulating factor (G-CSF) for treating severe chronic neutropenia (SCN) in a patient in need thereof, wherein the patient does not have a gain-of-function mutation in the CXCR4 gene and does not have a genetic abnormality associated with WHIM syndrome, said use being defined in the claims.

In some embodiments, the patient has an absolute neutrophil count (ANC) less than about 500 cells/µL.

In some embodiments, G-CSF is co-administered to the patient at a starting dosage of about 6 mcg/kg as a twice daily subcutaneous injection (for a patient having congenital neutropenia); or about 5 mcg/kg as a single daily subcutaneous injection (for a patient having idiopathic or cyclic neutropenia). In some embodiments, the patient is already receiving G-CSF and continues chronic dosing at a dosage sufficient to maintain clinical benefits, such as daily administration in the amount of about 6 mcg/kg (for patients having congenital neutropenia); about 2.1 mcg/kg (for patients having cyclic neutropenia); or about 1.2 mcg/kg (for patients having idiopathic neutropenia).

For treatment of severe, chronic neutropenia, Neupogen^{®} (filgrastim or G-CSF) is indicated at a starting dosage 6 mcg/kg as a twice daily subcutaneous injection (congenital neutropenia); or 5 mcg/kg as a single daily subcutaneous injection (idiopathic or cyclic neutropenia). It is further indicated that the starting dosage by followed by chronic daily administration in order to maintain clinical benefits. The indicated chronic daily administration is in the amount of 6 mcg/kg (congenital neutropenia); 2.1 mcg/kg (cyclic neutropenia); and 1.2 mcg/kg (idiopathic neutropenia). Neulasta^{®} (pegfilgrastim or pegylated G-CSF) is not presently approved for treatment of severe, chronic neutropenia other than in patients receiving myelosuppressive chemotherapy or radiation. It is available in a 6 mg/0.6 mL single-dose prefilled syringe, which may be administered once per chemotherapy cycle, or in two doses of 6 mg each, one week apart, for subjects who have been exposed to radiation levels in excess of 2 gray (Gy). Neulasta^{®} is also available for use with the "on-body injector" or OBI, which is co-packaged with a prefilled syringe, and which administers the Neulasta^{®} dose over a period of approximately 45 minutes, beginning approximately 27 hours after the OBI is applied to the subject's skin.

In some embodiments, the effective amount of G-CSF is less than the approved dosage as a monotherapy for a similar patient being treated with the G-CSF.

In some embodiments, the present invention features a decrease in the incidence of bone pain in the patient, or across a representative group of patients. In some embodiments, the present invention features a decrease in the incidence of flu-like symptoms in the patient, or across a representative group of patients. In some embodiments, the present invention features a decrease in the incidence of a myeloid malignancy, such as myelodysplasia (MDS) or acute myeloid leukemia (AML), in the patient, or across a representative group of patients. Bone pain is estimated to occur in anywhere from 24% and (reported on filgrastim and pegfilgrastim labels, respectively) to as high as 66% for filgrastim [Ferguson (2015), Practical Pain Management, vol. 15 online at: practical painmanagement.com/treatments/pharmacological/non-opioids/antihistamine-g-csf-induced-bone-pain] and 59% (24% severe bone pain) for pegfilgrastim (Kirshner et al. (2012) J. Clin Oncol. 30:1974-79). G-CSF is also associated with flu-like symptoms. Further, a link between G-CSF and myeloid malignancies, such as myelodysplasia (MDS) or acute myeloid leukemia (AML) has been reported.

It is anticipated by the inventors that administration of mavorixafor will permit reduction of the G-CSF for at least some patients. In some cases, this reduces the risk of G-CSF associated malignancy and myelofibrosis, and reduces G-CSF associated bone pain while maintaining protection from infection.

In some embodiments, the neutropenia is SCN. In some embodiments, the neutropenia is CIN. In some embodiments, the neutropenia is AIN. In some embodiments, the neutropenia is caused by an autoimmune disorder such as systemic lupus erythematosus (SLE).

In some embodiments, a provided method comprises administering the mavorixafor, or a pharmaceutically acceptable salt or composition thereof, to a patient in a fasted state.

In some embodiments, the mavorixafor is in the form of a free base. In some embodiments, the mavorixafor is in the form of a pharmaceutically acceptable salt.

The patient has previously been treated with G-CSF.

In some embodiments, the mavorixafor, or a pharmaceutically acceptable salt or composition thereof, and the G-CSF, or another granulocyte-colony stimulating factor treatment such as those described herein, act synergistically. In some embodiments, the synergism comprises a more effective treatment of the disease than with either agent alone. In some embodiments, the synergism comprises a lower dose of one or both agents providing effective treatment for the disease than would be the case if either agent were used alone.

In some embodiments, the patient is currently being treated with G-CSF. In some embodiments, the dose and/or frequency of administration of G-CSF (while maintaining effectiveness of the treatment regimen) is/are reduced after treatment with mavorixafor, or a pharmaceutically acceptable salt thereof, is commenced.

In some embodiments, the patient has idiopathic neutropenia. In some embodiments, the patient has severe idiopathic neutropenia. In some embodiments, the patient has chronic neutropenia. In some embodiments, the patient has SCN, CIN, or AIN. In some embodiments, the patient has undergone genetic testing but no diagnosis of a genetic abnormality has been made. In some embodiments, the genetic testing was inconclusive. In some embodiments, the genetic testing revealed no known genetic abnormality, or a genetic abnormality not associated with neutropenia. In some embodiments, the patient has neutropenia not due to a genetic abnormality and due to one or more of an infectious, inflammatory, autoimmune, or malignant cause. In some embodiments, the malignant cause is a cancer.

In some embodiments, the patient has severe congenital neutropenia.

In some embodiments, the patient has undergone genetic testing and a genetic abnormality other than one associated with WHIM syndrome (e.g., a gain-of-function mutation in the *CXCR4* gene) has been diagnosed. In some embodiments, the patient has a congenital neutropenia. In some embodiments, the patient has a genetic abnormality selected from GSD1b, G6PC3 deficiency, GATA2 deficiency, a genetically-defined condition without myeloid maturation arrest at the myelocyte/promyelocyte stage, or an undefined genetic abnormality.

In some embodiments, a provided method further comprises the step of obtaining a biological sample from the patient and measuring the amount of a disease-related biomarker. In some embodiments, the biological sample is a blood sample. In certain embodiments, the disease-related biomarker is selected from the group consisting of CXCR4, SDF-1α/CXCL12; and GRK3 (G protein coupled receptor kinase 3).

The dose level and regimen may be set by the treating clinician, and typically depends on factors such as the age, weight, sex, and general health of the patient. In some embodiments, mavorixafor, or a pharmaceutically acceptable salt thereof, is administered in an oral dose, such as PO QD, of from about 25 mg/day to about 1200 mg/day. In some embodiments, the daily dose is from about 50 mg/day to about 800 mg/day; from about 100 mg/day to about 800 mg/day; from about 150 mg/day to about 800 mg/day; from about 200 mg/day to about 800 mg/day; from about 250 mg/day to about 800 mg/day; from about 300 mg/day to about 800 mg/day; from about 350 mg/day to about 800 mg/day; or from about 400 mg/day to about 800 mg/day.

In some embodiments, the daily dose is from about 100 mg/day to about 600 mg/day; from about 200 mg/day to about 600 mg/day; from about 300 mg/day to about 500 mg/day; or from about 350 mg/day to about 450 mg/day. In a particular embodiment, mavorixafor or a pharmaceutically acceptable salt thereof is administered in a daily dose of about 400 mg/day PO QD. Although the daily dose is preferably administered once daily, the clinician may also choose to divide the dose into two or more parts taken at intervals during the day. For example, a daily dose may be divided into two parts, with one half of the daily dose administered in the morning, and the second half of the daily dose administered in the afternoon or evening. The interval between halves of the daily dose may be from 4 hours to about 16 hours; preferably from about 5 hours to about 15 hours; or more preferably from about 6 hours to about 14 hours; from about 7 hours to about 13 hours; or from about 8 hours to about 12 hours.

In some embodiments, cells taken from the patient exhibit increased expression of CXCR4.

In some embodiments, the method further comprises the step of obtaining a biological sample from the patient and measuring the amount of a disease-related biomarker.

In some embodiments, the biological sample is a blood sample.

In some embodiments, the disease-related biomarker is ANC, ALC, total White Blood Cell counts (WBC), or circulating CXCR4.

In some embodiments, the mavorixafor or a pharmaceutically acceptable salt or composition thereof is administered orally (PO) once per day (QD).

In some embodiments, the mavorixafor or a pharmaceutically acceptable salt or composition thereof is administered orally (PO) twice per day (BID).

In some embodiments, a disclosed method comprises administering a mavorixafor unit dosage form comprising a composition comprising:
(a) mavorixafor, or a pharmaceutically acceptable salt thereof, as about 10-20% by weight of the composition;
(b) microcrystalline cellulose as about 70-85% by weight of the composition;
(c) croscarmellose sodium as about 5-10% by weight of the composition;
(d) sodium stearyl fumarate as about 0.5-2% by weight of the composition; and
(e) colloidal silicon dioxide as about 0.1-1.0% by weight of the composition.

In some embodiments, the unit dosage form is in capsule form.

In some embodiments, the dosage form comprises about 25 mg mavorixafor, or a pharmaceutically acceptable salt thereof. In other embodiments, the dosage form comprises about 50 mg; 100 mg; 200 mg; 300 mg; 400 mg; 500 mg; 600 mg; or 800 mg mavorixafor, or a pharmaceutically acceptable salt thereof.

In some embodiments, the present invention provides a method for treating neutropenia, such as SCN or CIN, in a patient in need thereof, comprising the step of administering to the patient a disclosed unit dosage form.

In some embodiments, the present invention provides a method for treating neutropenia, such as SCN or CIN, in a patient in need thereof, comprising administering to said patient mavorixafor, or a pharmaceutically acceptable salt or composition thereof, in an amount effective to increase absolute neutrophil count (ANC) and/or to increase absolute lymphocyte count (ALC) in the patient, for example in the patient's blood. In some embodiments, the ANC and/or ALC is increased in the patient by at least 10%, 15%, 20%, 25%, 30%, 35%, 40%, 45% or at least 50% of that of the pre-treatment baseline counts.

In some embodiments, the present invention provides a method for treating neutropenia, such as SCN or CIN, in a patient in need thereof, comprising administering to said patient mavorixafor or a pharmaceutically acceptable salt or composition thereof, in an amount effective to increase absolute neutrophil count (ANC) to a level greater than or equal to 500/µL and/or to increase absolute lymphocyte count (ALC) to a level greater than or equal to 1000/µL.

In some embodiments, said patient originally exhibits ANC less than 600/µL and/or ALC less than 1000/µL before treatment with mavorixafor, or a pharmaceutically acceptable salt or composition thereof.

In some embodiments, said patient originally exhibits ANC less than 500/µL and/or ALC less than 650/µL before treatment with mavorixafor or a pharmaceutically acceptable salt or composition thereof.

In some embodiments, a disclosed method results in increases in ANC levels to at least about 500/µL, at least about 600/µL, at least about 700/µL, at least about 800/µL, at least about 900/µL ,at least about 1000/µL, at least about 1,100/µL, or at least about 1,200/µL, or to about that of a human with a normally-functioning immune system, on at least 85% of assessments.

In some embodiments, a disclosed method results in increases in ALC to at least about 1000/µL, about 1,200/µL, or about 1,500/µL, or to about that of a human with a normally-functioning immune system, on at least 85% of assessments.

In some embodiments, a disclosed method results in a lowered frequency of infections in the patient, such as at least 10%; at least 25%; or at least 50% less infections. In some embodiments, the method reduces the frequency of a respiratory tract infection.

In some embodiments, a disclosed method results in increased levels of total circulating WBC, neutrophils, and/or lymphocytes. In some embodiments, cell counts of WBC, neutrophils, and/or lymphocytes increase to approximately 1.4 x baseline. In some embodiments, cell counts of WBC, neutrophils, and/or lymphocytes increase to approximately 1.6 x baseline, 1.8 x baseline, or 2.0 x baseline. In some embodiments, cell counts of WBC, neutrophils, and/or lymphocytes increase to approximately 2.9 x baseline. In some embodiments, cell counts of lymphocytes increase to approximately 2.9 x baseline. In some embodiments, cell counts of neutrophils increase to approximately 2.7 x baseline and lymphocytes to approximately 1.9 x baseline.

In some embodiments, the present invention provides a method of treating neutropenia, such as SCN or CIN, in a patient in need thereof, wherein said patient has been receiving regular or preventative treatment with G-CSF. The method comprises administering to said patient an effective amount of mavorixafor. The timing of administration of mavorixafor may be prior to, together with, or subsequent to administration of G-CSF.

In certain embodiments, after commencement of administration of mavorixafor, the dosage of G-CSF administered to said patient may be reduced, while maintaining absolute neutrophil counts (ANC) equal to or higher than 500 cells/µL.

According to the present invention, the dosage of G-CSF that is administered to the patient is reduced by at least about 25% relative to the patient's previous dose before beginning treatment with mavorixafor or a pharmaceutically acceptable salt or composition thereof. In certain embodiments, the dosage of G-CSF that is administered to the patient is reduced by at least about 50%, 75%, or 95% relative to the patient's previous dose before beginning treatment with mavorixafor or a pharmaceutically acceptable salt or composition thereof. In certain embodiments, the dosage of G-CSF that is administered to the patient is reduced by about 25%, 30%, 35%, 40%, 50%, 55%, 60%, 65%, 70%, 75%, 80%, 85%, 90%, or 95%.

In certain embodiments, the frequency of dosage of G-CSF is reduced, for example, reduced in frequency by at least 25%, 50%, 75%, or 90%.

Decreased dosage of G-CSF can be effected by lowering the doses administered at any one time and/or by increasing the interval between dosage administration, e.g., once every three days, rather than once every two days.

In some embodiments, the patient begins with a well-tolerated dose of oral, daily mavorixafor, for example, 400 mg per day, wherein the patient is presently receiving a full dose (1X) of G-CSF or peg-G-CSF. The patient is typically monitored for ANC. In some embodiments, if the patient's ANC is at or above 1000 cells/µL, patient's dose of G-CSF or peg-G-CSF is reduced by a factor of approximately 25%, (i.e., to 0.75X dose). In some embodiments, if ANC remains at or above 1000 cells/µL, then (a) the patient's dose of G-CSF or peg-G-CSF is further reduced; (b) the daily dosage of mavorixafor being administered is increased or decreased; or both (a) and (b). Typically, at such time, ANC will continue to be monitored, with a goal of ANC of at least 500 cell/µL being maintained. As long as the patient's ANC remains above 500 cells/µL, the patient's dose of G-CSF or peg-G-CSF is optionally further reduced. In some embodiments, the method reduces bone pain or other adverse effects of G-CSF or peg-G-CSF.

If the patient's measured ANC is found to be between 500 and 1000 cells/µL, (a) the patient's dose of G-CSF or peg-G-CSF is further reduced; (b) the daily mavorixafor dosage is increased; or both (a) and (b). In some embodiments, the method provides maintenance of an ANC of at least 500 cell/µL. In some embodiments, as long as the patient's ANC remains above 500 cells/µL, the patient's dose of G-CSF or peg-G-CSF is optionally further reduced. In some embodiments, the method reduces bone pain or other adverse effects of G-CSF or peg-G-CSF.

### Dosage and Formulations

### Mavorixafor

CXCR4 inhibitors such as the compound mavorixafor (previously known as X4P-001, AMD070, or AMD11070) or a pharmaceutically acceptable salt thereof or pharmaceutical composition thereof, as described in greater detail below, are useful both as a monotherapy and as a combination therapy with one or more other therapeutic agents described herein. Accordingly, in one aspect, the present invention provides a method of treating neutropenia, such as those described herein, by administering to a patient in need thereof an effective amount of a CXCR4 inhibitor such as mavorixafor, or a pharmaceutically acceptable salt thereof or pharmaceutical composition thereof. The method according to the invention further includes co-administering simultaneously or sequentially an effective amount of one or more additional therapeutic agents, such as those described herein.

Mavorixafor (formerly known as X4P-001, AMD 070, or AMD11070) is a small molecule antagonist of CXCR4 having the potential to block the enhanced signaling activity of wild type and mutant CXCR4, resulting in an increase in the number of circulating white blood cells (Leukocytosis) of 2.9-fold (400-mg single-dose subject) above baseline with a peak between 2 and 4 h following dosing (Stone, 2007) by inhibiting CXCR4-dependent interactions between bone marrow stromal cells and mature leukocytes of many lineages thus allowing release of these cells into the circulation (Liles Blood 2003).

Mavorixafor is a second-generation, small-molecule, non-competitive, allosteric antagonist of chemokine receptor type 4 (CXCR4) that acts by binding to extracellular domains of the receptor, resulting in specific and reversible inhibition of receptor signaling in response to its ligand C-X-C motif chemokine ligand 12 (CXCL12). Mavorixafor is currently in clinical development in patients with cancer (renal cell carcinoma), Waldenström Macroglobulinemia, and with warts, hypogammaglobulinemia, infections, and myelokathexis (WHIM) syndrome. The chemical formula is: C₂₁H₂₇N₅; and molecular weight is 349.48 amu. The chemical structure of mavorixafor is as follows according to Formula I:

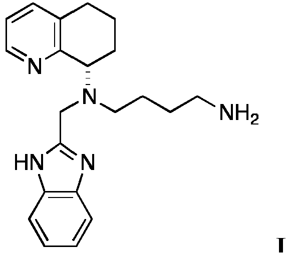

As of May 2019, approximately 193 healthy volunteers and patients had been treated with mavorixafor in clinical studies (n= 70 healthy volunteers, n= 16 HIV, n= 99 oncology, n=8 WHIM syndrome). Overall, mavorixafor has been generally well tolerated, with no mavorixafor-related serious AEs (SAEs) causing a fatal outcome in any of the patients.

In certain embodiments, the mavorixafor, pharmaceutically acceptable salt thereof, or composition comprising mavorixafor or a pharmaceutically acceptable salt thereof is administered orally (PO) once daily (QD) or twice daily (BID), in an amount from about 25 mg to about 800 mg daily. In certain embodiments, the dosage composition may be provided twice a day in divided dosage, approximately 12 hours apart. In other embodiments, the dosage composition may be provided once daily. The terminal half-life of mavorixafor has been generally determined to be between about 12 to about 24 hours, or approximately 14.5 hrs. In certain embodiments, the dosage of mavorixafor useful in the invention is from about 25 mg to about 1200 mg daily. In other embodiments, the dosage of mavorixafor useful in the invention may range from about 25 mg to about 1000 mg daily, from about 50 mg to about 800 mg daily, from about 50 mg to about 600 mg daily, from about 50 mg to about 500 mg daily, from about 50 mg to about 400 mg daily, from about 100 mg to about 800 mg daily, from about 100 mg to about 600 mg daily, from about 100 mg to about 500 mg daily, from about 100 mg to about 400 mg daily; from about 200 mg to about 800 mg daily, from about 200 mg to about 600 mg daily, from about 300 mg to about 600 mg daily, from about 200 mg to about 500 mg daily from about 200 mg to about 400 mg daily.

In other embodiments, the dosage of mavorixafor or a pharmaceutically acceptable salt thereof is administered in a dosage range from about 100 mg to about 800 mg daily, from about 200 mg to about 600 mg daily, from about 300 mg to about 500 mg daily, or from about 350 mg to about 450 mg daily; or in a daily dosage of about 100 mg/day; 125 mg/day; 150 mg/day; 175 mg/day; 200 mg/day; 225 mg/day; 250 mg/day; 275 mg/day; 300 mg/day; 325 mg/day; 350 mg/day; 400 mg/day; 425 mg/day; 450 mg/day; 475 mg/day; 500 mg/day; 525 mg/day; 550 mg/day; 575 mg/day; 600 mg/day; 625 mg/day; 650 mg/day; 675 mg/day; 700 mg/day; 725 mg/day; 750 mg/day; 775 mg/day or 800 mg/day. In unusual cases, the dosage of mavorixafor or a pharmaceutically acceptable salt thereof may be administered in an amount in excess of 800 mg/day, while taking care to minimize or avoid any adverse effects of such administration.

In some embodiments, a provided method comprises administering to the patient a pharmaceutically acceptable composition comprising mavorixafor wherein the composition is formulated for oral administration. In certain embodiments, the composition is formulated for oral administration in the form of a tablet, a caplet or a capsule. In some embodiments, the composition comprising mavorixafor is formulated for oral administration in the form of a capsule.

In certain embodiments, a provided method comprises administering to the patient one or more dosage forms comprising 25 mg to 1200 mg mavorixafor active ingredient; and one or more pharmaceutically acceptable excipients. In certain embodiments, the capsule is comprised of hard gelatin. In some embodiments the dosage form comprises 25 mg to 800 mg mavorixafor active ingredient, 50 mg to 600 mg mavorixafor active ingredient, 100 mg to 500 mg mavorixafor active ingredient, 100 mg to 400 mg mavorixafor active ingredient, 100 mg to 300 mg mavorixafor active ingredient, or 100 mg to 200 mg mavorixafor active ingredient.

In certain embodiments, a disclosed method comprises administering a composition comprising mavorixafor, or a pharmaceutically acceptable salt thereof, one or more diluents, a disintegrant, a lubricant, a flow aid, and a wetting agent. In some embodiments, a disclosed method comprises administering a composition comprising 25 mg to 1200 mg mavorixafor, or a pharmaceutically acceptable salt thereof, microcrystalline cellulose, dibasic calcium phosphate dihydrate, croscarmellose sodium, sodium stearyl fumarate, colloidal silicon dioxide, and sodium lauryl sulfate. In some embodiments, a disclosed method comprises administering a unit dosage form wherein said unit dosage form comprises a composition comprising 25 mg to 200 mg mavorixafor, or a pharmaceutically acceptable salt thereof, microcrystalline cellulose, dibasic calcium phosphate dihydrate, croscarmellose sodium, sodium stearyl fumarate, colloidal silicon dioxide, and sodium lauryl sulfate. In certain embodiments, a disclosed method comprises administering a unit dosage form comprising a composition comprising mavorixafor, or a pharmaceutically acceptable salt thereof, present in an amount of about 25 mg, about 40 mg, about 50 mg, about 80 mg, about 100 mg, about 150 mg, about 200 mg, about 250 mg, about 300 mg, about 350, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, or about 1200 mg. In some embodiments, a provided composition (or unit dosage form) is administered to the patient once per day, twice per day, three times per day, or four times per day. In some embodiments, a provided composition (or unit dosage form) is administered to the patient once per day or twice per day.

In some embodiments, a disclosed method comprises administering a unit dosage form comprising a composition comprising:
(a) mavorixafor, or a pharmaceutically acceptable salt thereof, as about 10-30% by weight of the composition;
(b) microcrystalline cellulose as about 60-80% by weight of the composition;
(c) croscarmellose sodium as about 5-10% by weight of the composition;
(d) sodium stearyl fumarate as about 0.5-2% by weight of the composition; and
(e) colloidal silicon dioxide as about 0.1-1.0 % by weight of the composition.

In some embodiments, a disclosed method comprises administering a unit dosage form comprising a composition comprising:
(a) mavorixafor, or a pharmaceutically acceptable salt thereof, as about 15% by weight of the composition;
(b) microcrystalline cellulose as about 78% by weight of the composition;
(c) croscarmellose sodium as about 6% by weight of the composition;
(d) sodium stearyl fumarate as about 1% by weight of the composition; and
(e) colloidal silicon dioxide as about 0.2% by weight of the composition.

In some embodiments, a disclosed method comprises administering a unit dosage form comprising a composition comprising:
(a) mavorixafor, or a pharmaceutically acceptable salt thereof, as about 10-20% by weight of the composition;
(b) microcrystalline cellulose as about 25-40% by weight of the composition;
(c) dibasic calcium phosphate dihydrate as about 35-55% by weight of the composition;
(d) croscarmellose sodium as about 4-15% by weight of the composition;
(e) sodium stearyl fumarate as about 0.3-2% by weight of the composition;
(f) colloidal silicon dioxide as about 0.1-1.5% by weight of the composition; and
(g) sodium lauryl sulfate as about 0.1-1.5% by weight of the composition.

In some embodiments, a disclosed method comprises administering a unit dosage form comprising a composition comprising:
(a) mavorixafor, or a pharmaceutically acceptable salt thereof, as about 13% by weight of the composition;
(b) microcrystalline cellulose as about 32% by weight of the composition;
(c) dibasic calcium phosphate dihydrate as about 44% by weight of the composition;
(d) croscarmellose sodium as about 8% by weight of the composition;
(e) sodium stearyl fumarate as about 1.4% by weight of the composition;
(f) colloidal silicon dioxide as about 0.4% by weight of the composition; and
(g) sodium lauryl sulfate as about 0.7% by weight of the composition.

In some embodiments, a disclosed method comprises administering a unit dosage form comprising a composition comprising:
(a) mavorixafor, or a pharmaceutically acceptable salt thereof, as about 35-75% by weight of the composition;
(b) microcrystalline cellulose as about 5-28% by weight of the composition;
(c) dibasic calcium phosphate dihydrate as about 7-30% by weight of the composition;
(d) croscarmellose sodium as about 2-10% by weight of the composition;
(e) sodium stearyl fumarate as about 0.3-2.5% by weight of the composition;
(f) colloidal silicon dioxide as about 0.05-1.2% by weight of the composition; and
(g) sodium lauryl sulfate as about 0.2-1.2% by weight of the composition.

Inasmuch as it may be desirable to administer a combination of active compounds, for example, for the purpose of treating a particular disease or condition, it is within the scope of the present invention that two or more pharmaceutical compositions, at least one of which contains a compound in accordance with the invention, may conveniently be combined in the form of a kit suitable for co-administration of the compositions. Thus, the kit includes two or more separate pharmaceutical compositions, at least one of which contains a compound of the invention, and means for separately retaining said compositions, such as a container, divided bottle, or divided foil packet. An example of such a kit is the familiar blister pack used for the packaging of tablets, capsules and the like.

The kit is particularly suitable for administering different dosage forms, for example, oral and parenteral, for administering the separate compositions at different dosage intervals, or for titrating the separate compositions against one another. To assist compliance, the kit typically includes directions for administration and may be provided with a memory aid.

The examples below explain the invention in more detail. The following preparations and examples are given to enable those skilled in the art to more clearly understand and to practice the present invention.

### EXEMPLIFICATION

### Example 1: Non-Clinical Evaluation of X4P-001 Effects on CXCR4: In Vitro Pharmacology

The in-vitro pharmacology of X4P-001 (formally designated AMD11070) was extensively studied and the results reported [Mosi 2012]. Presented below is the relevant information from the Mosi 2012 literature publication. The SDF-1α isoform was used for the experiments described below.

### X4P-001 Inhibition of SDF-1α Binding to CXCR4

X4P-001 was shown to inhibit binding of [125I]-SDF-1α to CCRF-CEM cells (T-lymphoblastoid cell line which naturally express CXCR4 [Crump 1997]) in a heterologous competition binding assay. The results of the assay are shown in **FIG. 4****.** The data was fitted to a single site binding model and gave an IC50 of 12.5 ± 1.3 nM.

### X4P-001 Inhibition of CXCR4 Cell Signaling

CXCR4 is a G-protein coupled receptor [Baggiolini 1998, Zlotnik 2000]. As such the activation of the receptor can be measured using a nonhydrolysable analogue of GTP such as fluorescently labeled Europium-GTP (Eu-GTP) or radiolabeled [35S]-GTPγS. The results shown in **FIG. 5** and **FIG. 6** showed that X4P-001 inhibited CXCR4 activation with IC50 values of 39.8 ± 2.5 nM and 19.0 ± 4.1 nM in the Eu-GTP binding and [³⁵S]-GTPγS assays, respectively.

Upon activation of a G-protein coupled receptor, intracellular signaling pathways are triggered resulting in the release of calcium from intracellular stores. This calcium flux can be assayed using a calcium-chelating molecule, Fluo-4, which fluoresces upon binding calcium. X4P-001 was able to inhibit SDF-1α (2.5 nM SDF-1α) mediated calcium flux in CCRF-CEM cells with an IC50 of 9.0 ± 2.0 nM. The result is shown in **FIG. 7****.**

A key property of all chemokines is that they induce a chemotactic response to a chemokine concentration gradient. X4P-001 was able to inhibit SDF-1α mediated chemotaxis of CCRF-CEM cells with an IC50 of 19.0 ± 4.0 nM as shown in **FIG. 8****.**

A summary of the above in vitro results is presented in **Table** 2 below:

**Table 2: In Vitro Concentrations of X4P-001(IC50) Associated with Different Biological Responses**

| **Response** | **IC₅₀ (nm)** |
|---|---|
| Ligand Binding | 12.5 ± **1.3** |
| Eu-GTP | 39.8 ± 2.5 |
| [³⁵S]-GTP | 19.0 ± 4.1 |
| Calcium Flux | 9.0 ± 2.0 |
| Chemotaxis | 19.0 ± 4.0 |
| Average IC₅₀ | 21.5 |

### Mavorixafor Selectivity for CXCR4

In order to demonstrate the specificity of X4P-001 for CXCR4 it was tested in calcium signaling assays against a panel of chemokine receptors, and in ligand binding assays for BLT1, the receptor for leukotriene B4 (LTB4), and CXCR7. LTB4 is a potent chemoattractant and its receptor is a G-protein coupled receptor. The results in Table 3 show that the IC₅₀ of X4P-001 against CCR1, CCR2b, CCR4, CCR5, CCR7, CXCR3, and LTB4 was >50 mM in all cases. X4P-001 did not inhibit SDF-1α binding to CXCR7 at a concentration of 10 mM, the maximum concentration tested in this assay. Together, these data indicate that X4P-001 is a selective inhibitor of CXCR4.

**Table 3: Calcium Flux Response for Cell Lines Treated with Mavorixafor for IC50 Determination**

| **Receptor** | **Cell line** | **Ligand** | **IC₅₀ Mavorixafor (µM)** |
|---|---|---|---|
| **CCR1** | **HEK293F-CCR1** | **MIP-1α/CCL3** | **>50** |
| **CCR2b** | **HEK293F-CCR2b** | **MCP-1/CCL2** | **>50** |
| **CXCR3** | **HEK293F-CXCR3-Gαq15** | **IP-10/CXCL10** | **>50** |
| **CXCR7** | **Cf2Th.CXCR7** | **SDF-1α/CXCL12** | **>10** |
| **CCR4** | **HEK293F-CCR4-Gαq15** | **TARC/CCL17** | **>50** |
| **CCR5** | **HEK293F-CCR5** | **RANTES/CCL5** | **>50** |
| **CCR7** | **CCRF-CEM** | **MIP-3β/CCL19** | **>50** |
| **BLT₁** | **CHO-S-LTB₄** | **LTB₄** | **>50** |

### Discussion and Conclusions from In Vitro Studies

Using the CCRF-CEM cell line, which naturally expresses CXCR4 [Crump 1997] it was shown that X4P-001 inhibits SDF-1α ligand binding to CXCR4 with an IC50 of 12.5 ± 1.3 nM. X4P-001 also inhibited CXCR4 activation and signaling as shown by inhibition of SDF-1α mediated G-protein activation of the CXCR4 receptor in two assays using either the fluorescent Eu-GTP or the radiolabeled [³⁵S]-GTPγS binding assays with IC50 values of 39.8 ± 2.5 nM and 19.0 ± 4.1 nM, respectively, and inhibition of SDF-1α mediated calcium flux with an IC50 of 9.0 ± 2.0 nM. X4P-001 also inhibited SDF-1α-mediated chemotaxis, a CXCR4-mediated physiological response, with an IC50 of 19.0 ± 4.0 nM. In addition, X4P-001 had little or no inhibitory effect on either MIP1α, MCP-1, TARC, RANTES, MIP-3β, or IP10 mediated calcium flux, ligands for CCR1, CCR2b, CCR4, CCR5, CCR7 and CXCR3, respectively, or SDF-1α binding to CXCR7, or LTB4 binding to BLT1, an alternative G-protein coupled receptor that mediates chemotaxis. These data indicate that X4P-001 is a selective inhibitor of CXCR4 over the other chemokine receptors evaluated.

Additionally, it was shown that X4P-001 is an allosteric inhibitor of CXCR4 by comparing the dose/response of SDF-1α in the calcium flux assay in the presence of increasing amounts of X4P-001 [Mosi 2012]. Based on inhibition being mediated by non-competitive binding, the extent of inhibition is therefore dependent solely on the concentration of X4P-001 and is independent of the concentration of SDF-1α ligand.

### In-Vivo Pharmacology

The primary in vivo pharmacologic effect of X4P-001 is mobilization of white blood cells (WBC) from bone marrow. Three studies are summarized below which demonstrate the mobilization of WBC from the bone marrow of beagle dogs and C3W/He J mice.

### Hematologic Effects in the Male Beagle Dog

Three fasted male Beagle dogs received a single dose of X4P-001 in aqueous solution by oral gavage at dose levels of 5, 15, and 35 mg/kg (1 dog per dose level) in a volume of 1mL/kg. Blood samples (approximately 3mL each) were obtained at multiple timepoints from each animal by direct venipuncture of the jugular vein and collected using Vacutainer^{®} tubes containing K3EDTA as the anticoagulant. Blood samples were obtained at pre-dose, and 0.25, 0.5, 0.75, 1, 1.5, 2, 3, 4, 7, 12, and 24 hours post-dose. Blood samples were stored at ambient room temperature prior to automated differential analysis.

Body weights were determined prior to dosing on the day of test article administration. Animals were observed at least once daily and at times of blood sampling.

Hematology parameters included the following:
- White Blood Cell Count (WBC)
- Differential white blood cell count (absolute and relative)
- Neutrophil
- Lymphocytes
- Monocytes
- Eosinophils
- Basophils
- Large Unstained Cells (LUC)
- Hematocrit (HCT)
- Hemoglobin (HGB)
- Mean Corpuscular Hemoglobin (MCH)
- Mean Corpuscular Hemoglobin Concentration (MCHC)
- Mean Corpuscular Volume (MCV)
- Platelet Count (PLT)
- Red Blood Cell Count (RBC)

### Results

The effect of X4P-001 on WBC and absolute neutrophil and lymphocyte counts is shown in **FIG. 8****.** Maximal increases in WBC occurred 4-12 hours post-dose. Peak elevations ranged from 1.8-2.9-fold above baseline values at the 15 and 35 mg/kg dose levels, with somewhat lower (1.5-fold) elevations observed at the 5 mg/kg dose level. Although limited by the small sample size, these results suggest that maximal increases may have been achieved at the higher dose levels. WBC, neutrophil, and lymphocyte counts remained elevated at the 15 and 35 mg/kg dose levels at 24 hours, with evidence of return to baseline. No other hematological effects were observed.

### A 28-Day Oral (Capsule) Study in the Beagle Dog with a 14-Day Recovery Period

A 28-Day GLP oral (capsule) toxicology study was conducted with X4P-001 in the male and female beagle dog, and hematology effects were observed, with X4P-001 administered twice-daily (at least 7 hours apart) by oral capsule for 28 days. A subset of treated animals was evaluated after a 14-day recovery period. Table 4 presents the protocol design and Table 5 the evaluations schedule.

**Table 4: Protocol Design for 28-Day Toxicity Study in the Dog**

| **Group** | **Dose Level (mg/kg/day)^{a}** | **Animals Terminal Necropsy** | **Animals 14-day Recovery** |
|---|---|---|---|
| 1 | 0 (empty capsule) | 3 M, 3 F | 2 M, 2 F |
| 2 | 10 | 3 M,3 F | -- |
| 3 | 30 | 3 M,3 F | -- |

**Table 5: Protocol Evaluations and Schedules**

| **Evaluations** | **Schedule** |
|---|---|
| Study Duration | Days -10 through Day 42 |
| Treatment | Days 1 through 28, twice daily |
| Clinical Observation | Twice Daily |
| Food Consumption | Daily |
| Body Weight | Weekly |
| Vital Signs^{a} | Predose acclimation period; final dosing week; final recovery week |
| Ophthalmology | Predose and during Week 4 |
| Electrocardiogram Evaluation | Predose and during Week 4, at ~1 hour post-first daily dose |
| Clinical Pathology^{b} | Predose d-10, d-2; Post-dose, Day 29 (all groups), Day 42 (recovery only) |
| Necropsy^{c} | Day 29, terminal; Day 42, recovery |

| | |
|---|---|
| a. Vital signs comprise heart rate, blood pressure, and body temperature b. Clinical pathology comprised hematology, coagulation, serum, and urinalysis (done only once predose). c. Necropsy studies comprise organ weight, macroscopic, and microscopic observations, including 500-cell bone marrow differential count. | |

As shown in Table 6 below, increases in absolute counts for neutrophils, lymphocytes, and monocytes were observed at termination (Day 28); these were of greater magnitude and more likely statistically significant in females. These changes were considered consistent with the pharmacological effects of X4P-001. After the 14-day recovery period (only 100 mg/kg dose group evaluated) all hematology results returned to within normal levels.

**Table 6: Hematology Findings at Termination in 28-Day Oral Toxicity Study in the Dog**

| **Observation** | **10 mg/kg/d (3 M, 3 F)** | **30 mg/kg/d (3 M, 3 F)** | **100 mg/kg/d (3 M, 3 F)** |
|---|---|---|---|
| Hematology | | | |
| Neutrophils (abs) | M incr 1.2x; F incr 1.9x† | M incr 1.2x; F incr 2.3x† | M incr 1.8x; F incr 2.8x† |
| Lymphocytes (abs) | M incr 1.3x; F incr 1.4x | M incr 1.6x; F incr 1.6x† | M incr 2.3x†; F incr 1.4x† |
| Monocytes (abs) | M incr 1.2x; F incr 1.6x† | M incr 1.3x; F incr 1.9x† | M incr 1.9x†; F incr 2.4x† |
| **Reticulocytes** | No changes | No changes | F decr 0.24† |
| **Coagulation** | No changes | No changes | No changes |

| | | | |
|---|---|---|---|
| abs, absolute; † p<0.05 compared with control animals of the same sex | | | |

### Hematologic Effects of X4P-001 in Mice

A further study was conducted to determine whether X4P-001 mobilizes progenitor/stem cells in mice. All experiments were performed in C3W/He J mice. X4P-001 and AMD3100/plerixafor were administered via single subcutaneous injection at the doses described below. The mobilization capacity of X4P-001 was assessed by the numbers of granulocyte-macrophage (CFU-GM), erythroid (BFU-E) and multipotential (CFU-GEMM) progenitor cells per mL of blood. The progenitors were stimulated to form colonies in vitro with the combination of lU/mL rhu EPO, 50 ng/mL rmu SLF, 5% vol/vol pokeweed mitogen mouse spleen cell conditioned medium (PWMSCM), and 0. 1 mM hemin. Plates were scored 7 days after incubation at 37°C, 5% CO2, lowered (5% CO2) and in a humidified chamber.

### Results

X4P-001 mobilized progenitors in C3H/HeJ mice following a single subcutaneous injection. In the first experiment (data shown in Table 7), mice received a dose of 5 mg/kg- and the number of progenitors in the circulating blood was measured at various time points (0.25, 0.5, 1, 2, 6 and 24 hours). The peak of nucleated cell mobilization occurred at approximately 1-2 hours post-injection. Peak increases of CFU-GM, BFU-E and CFU-GEMM were 4.21 (30 min.), 2.49-2.54 (30-60 min.), and 2.58- 2.67 (30-60 min.)-fold, respectively over control (saline injection).

**Table 7: X4P-001 Time Course of Progenitor Mobilization**

| | | **Control** | **Mavorixafor Time Course (dose = 5 mg/kg)** | | | | | |
|---|---|---|---|---|---|---|---|---|
| | | | **@ - 15"** | **@ - 30"** | **@ - 60"** | **@-2'** | **@ - 6'** | **@ - 24'** |
| **Nucleated Cellularity (x 10⁶/mL)** | **Mean** | 4.35 | 5.10 | 6.14 | 6.92 | 8.29 | 5.66 | 4.31 |
| | **STD** | 0.14 | 1.09 | 1.20 | 0.57 | 0.55 | 0.28 | 0.82 |
| **PBL-LD** | **STE** | 0.08 | 0.63 | 0.69 | 0.33 | 0.32 | 0.16 | 0.47 |
| | **Fold Chg** | **1.00** | **1.17** | **1.41** | **1.59** | **1.90** | **1.30** | **0.99** |
| | **P** | 1.000 | 0.307 | 0.062 | 0.002 | 0.000 | 0.002 | 0.930 |
| **GM** | **Mean** | 302.3 | 785.1 | 1273.8 | 866.0 | 897.5 | 387.5 | 386.3 |
| | **STD** | 20.5 | 180.3 | 85.4 | 197.8 | 165.6 | 54.6 | 110.5 |
| | **STE** | 11.8 | 104.1 | 49.3 | 114.2 | 95.6 | 31.5 | 63.8 |
| | **Fold Chg** | **1.00** | **2.60** | **4.21** | **2.86** | **2.97** | **1.28** | **1.28** |
| | **P** | 1.000 | 0.010 | 0.000 | 0.008 | 0.003 | 0.065 | 0.265 |
| **BFU** | **Mean** | 92.5 | 148.8 | 230.4 | 235.1 | 165.3 | 99.9 | 84.6 |
| | **STD** | 30.9 | 27.1 | 70.2 | 68.2 | 47.5 | 17.8 | 44.4 |
| | **STE** | 17.8 | 15.6 | 40.5 | 39.4 | 27.4 | 10.3 | 25.7 |
| | **Fold Chg** | **1.00** | **1.61** | **2.49** | **2.54** | **1.79** | **1.08** | **0.92** |
| | **P** | 1.000 | 0.076 | 0.036 | 0.030 | 0.090 | 0.735 | 0.814 |
| **GEMM** | **Mean** | 38.6 | 65.6 | 99.6 | 103.1 | 68.9 | 37.6 | 37.7 |
| | **STD** | 10.6 | 17.6 | 24.2 | 20.3 | 23.7 | 16.0 | 20.6 |
| | **STE** | 6.1 | 10.2 | 14.0 | 11.7 | 13.7 | 9.3 | 11.9 |
| | **Fold Chg** | **1.00** | **1.70** | **2.58** | **2.67** | **1.78** | **0.97** | **0.98** |
| | **P** | 1.000 | 0.085 | 0.016 | 0.008 | 0.114 | 0.934 | 0.946 |

| | | | | | | | | |
|---|---|---|---|---|---|---|---|---|
| Animals per group = 3, control group = 1, total animals = 21 | | | | | | | | |

An X4P-001 dose-response was performed by measurement of the number of circulating progenitors in the blood at 1 hour post-injection at various doses (1.5, 2.5, 5, 10 and 20 mg/kg). As shown in Table 8, there appears to be an upper limit to the number of progenitors that can be mobilized with X4P-001, exemplified by the fold increases of CFU-GM. The numbers of CFU-GM in the circulating blood dose-dependently increased with peak fold increase of 6.0 - 7.7 over control at 5-20 mg/kg. Peak fold increases respectively of 2.3 and 3.8 for BFU-E and CFU-GEMM were noted at 10 mg/kg. At doses below 5 mg/kg X4P-001, the fold-increases in the numbers of BFU-E and CFU-GEMM were not statistically significant.

**Table 8: Dose Response in C3H/HeJ Mice**

| | | **Control** | **Mavorixafor (mg/kg)** | | | | |
|---|---|---|---|---|---|---|---|
| | | | **20** | **10** | **5** | **2.5** | **1.5** |
| **Nucleated Cellularity (x 10⁶/mL)** | **Mean** | 6.48 | 9.62 | 9.94 | 7.65 | 8.29 | 6.94 |
| | **STD** | 0.69 | 1.26 | 4.02 | 2.74 | 2.07 | 0.50 |
| **PBL-LD** | **STE** | 0.40 | 0.73 | 2.32 | 1.58 | 1.20 | 0.29 |
| | **Fold Chg** | **1.00** | **1.48** | **1.53** | **1.18** | **1.28** | **1.07** |
| | **P** | 1.000 | 0.019 | 0.216 | 0.514 | 0.225 | 0.406 |
| **GM** | **Mean** | 188.0 | 1314.2 | 1444.2 | 1119.8 | 626.5 | 428.0 |
| | **STD** | 51.8 | 262.0 | 939.8 | 1011.9 | 220.4 | 118.7 |
| | **STE** | 29.9 | 151.2 | 542.6 | 584.2 | 127.3 | 68.5 |
| | **Fold Chg** | **1.0** | **7.0** | **7.7** | **6.0** | **3.3** | **2.3** |
| | **P** | 1.000 | 0.002 | 0.082 | 0.186 | 0.028 | 0.033 |
| **BFU** | **Mean** | 114.4 | 261.4 | 268.1 | 181.6 | 144.8 | 143.8 |
| | **STD** | 5.6 | 35.8 | 61.4 | 58.6 | 79.3 | 47.1 |
| | **STE** | 3.2 | 20.7 | 35.5 | 33.8 | 45.8 | 27.2 |
| | **Fold Chg** | **1.0** | **2.3** | **2.3** | **1.6** | **1.3** | **1.3** |
| | **P** | 1.000 | 0.002 | 0.012 | 0.119 | 0.544 | 0.343 |
| **GEMM** | **Mean** | 58.4 | 145.0 | 224.4 | 141.0 | 78.3 | 53.3 |
| | **STD** | 45.5 | 50.5 | 60.7 | 34.4 | 8.1 | 8.9 |
| | **STE** | 26.3 | 29.2 | 35.0 | 19.8 | 4.7 | 5.1 |
| | **Fold Chg** | **1.0** | **2.5** | **3.8** | **2.4** | **1.3** | **0.9** |
| | **P** | 1.000 | 0.092 | 0.019 | 0.066 | 0.498 | 0.857 |

| | | | | | | | |
|---|---|---|---|---|---|---|---|
| Animals per group = 3, control group 1, total animals = 18 | | | | | | | |

A final experiment was performed to compare the progenitor cell mobilization capacity of X4P-001 and AMD3100/plerixafor. Both drugs were administered subcutaneously at a dose of 5 mg/kg, and the number of progenitors in the circulating blood were measured for AMD3100 at a single 1 hour time point (the peak of mobilization with AMD3100, data not shown) versus X4P-001 at 0.25, 0.5, 1 and 2 hours post-injection. As shown in Table 9 comparing the fold-increase in CFU-GM, BFU-E, and CFU-GEMM, AMD3100 caused respective maximum increases of 9.11, 3.12, and 4.35, whereas respective peaks of mobilization with X4P-001 were 3.56, 2.84 and 3.21.

**Table 9: Mavorixafor Time Course Compared to AMD3100/Plerixafor (Dose 5mg/kg)**

| | | **Control** | **AMD3100** | **Mavorixafor** | | | |
|---|---|---|---|---|---|---|---|
| | | | **@ - 60"** | **@ - 15"** | **@ - 30"** | **@ - 60"** | **@ - 2'** |
| **Nucleated Cellularity (x10⁶/mL)** | **Mean** | 6.23 | 10.08 | 8.04 | 8.28 | 7.34 | 9.71 |
| | **STD** | 2.16 | 2.13 | 1.30 | 0.94 | 0.69 | 1.29 |
| **PBL-LD** | **STE** | 1.25 | 1.23 | 0.75 | 0.54 | 0.40 | 0.74 |
| | **Fold Chg** | **1.00** | **1.62** | **1.29** | **1.33** | **1.18** | **1.56** |
| | **P** | 1.000 | 0.092 | 0.281 | 0.205 | 0.444 | 0.074 |
| **GM** | **Mean** | 214.1 | 1950.3 | 588.3 | 705.9 | 761.4 | 619.6 |
| | **STD** | 118.2 | 566.4 | 168.1 | 151.5 | 239.2 | 158.7 |
| | **STE** | 68.2 | 327.0 | 97.1 | 87.5 | 138.1 | 91.6 |
| | **Fold Chg** | **1.00** | **9.11** | **2.75** | **3.30** | **3.56** | **2.89** |
| | **P** | 1.000 | 0.007 | 0.034 | 0.011 | 0.024 | 0.024 |
| **BFU** | **Mean** | 66.5 | 207.7 | 188.9 | 151.9 | 144.3 | 108.5 |
| | **STD** | 39.6 | 35.4 | 55.0 | 23.8 | 47.5 | 43.0 |
| | **STE** | 22.9 | 20.4 | 31.7 | 13.8 | 27.4 | 24.8 |
| | **Fold Chg** | **1.00** | **3.12** | **2.84** | **2.29** | **2.17** | **1.63** |
| | **P** | 1.000 | 0.010 | 0.035 | 0.033 | 0.095 | 0.281 |
| GEMM | **Mean** | 31.8 | 138.5 | 93.8 | 79.0 | 102.2 | 62.4 |
| | STD | 2.6 | 18.1 | 21.1 | 34.5 | 50.5 | 34.9 |
| | STE | 1.5 | 10.5 | 12.2 | 19.9 | 29.1 | 20.1 |
| | **Fold Chg** | **1.00** | **4.35** | **2.95** | **2.48** | **3.21** | **1.96** |
| | **P** | 1.000 | 0.001 | 0.007 | 0.078 | 0.074 | 0.205 |

| | | | | | | | |
|---|---|---|---|---|---|---|---|
| **Animals per group = 3, control group = 1, total animals = 18** | | | | | | | |

### Conclusions from In Vivo Studies

Single oral doses of X4P-001 at 5, 15, and 35 mg/kg in beagle dogs resulted in increased levels of total circulating WBC, neutrophils, and lymphocytes. The increases were consistently apparent at 4 hours and typically peaked at 12 hours, occasionally earlier. At 5 mg/kg, all three cell counts increased to 1.47x baseline. At 15 mg/kg, neutrophils increased to 1.8x and lymphocytes to 2.9x; and at 35 mg/kg, neutrophils to 2.7x and lymphocytes to 1.9x.

In multiple-dose toxicity studies in dogs, hematological effects after 28 days were qualitatively and quantitatively consistent with the findings in the single dose study in beagle dogs.

In C3H/HeJ mice, X4P-001 dose-dependently increased the number of circulating progenitors up to a dose of 5-10 mg/kg s.c.

### Example 2: Clinical Protocol: Patients to be Treated

### Patients with Severe CIN or Selected Congenital Neutropenias and Treated with Prophylactic G-CSF

Patients who may be treated in the study described below include patients with either a severe form of CIN or selected congenital neutropenia disorders.

To be eligible for treatment with mavorixafor in the present study, patients with severe CIN must have a history of ANC < 500 cells/µL, lasting for more than 3 months at any time since diagnosis; and must have been diagnosed with severe CIN more than 12 months ago that is not attributable to medications, infectious, genetic, inflammatory, autoimmune, or malignant causes. In this particular trial, the patients must be currently treated with a prophylactic steady-state G-CSF regimen for >15 days before receiving the first dose of mavorixafor, must have normal cytogenetics on the most recent bone marrow biopsy/aspirate, if performed; and must have no associated thrombocytopenia nor anemia before G-CSF therapy initiation.

To be eligible for the specific clinical trial, patients with selected congenital neutropenia conditions, including GSD1b *(GSDlb; SLC37A4),* G6PC3 deficiency (*G6PC3*), or GATA2 deficiency (*GATA2*) may currently be receiving steady-state G-CSF dosing, or may not have been on G-CSF for >15 days. The patient must have documentation of his or her mutational status.

The primary objectives of the following experiments are to determine the safety and tolerability of mavorixafor in patients with severe CIN and selected congenital neutropenia disorders defined as follows:

Severe CIN will be defined in this protocol as patients presenting an **ANC of** < **500 cells/µL,** lasting more than 3 months and diagnosed more than **12 months ago,** and not attributable to drugs or a specific genetic, infectious, inflammatory, autoimmune, or malignant cause.

Congenital neutropenia conditions that may be treated in accordance with the present study include the following:
a. **GSD1b** due to mutations in ***SLC37A4,***
b. **G6PC3** deficiency due to mutations in ***G6PC3,*** and
c. **GATA2** deficiency due to mutations in ***GATA2.***

Eligible patients with GSD1b, G6PC3 deficiency, or GATA2 deficiency must be ≥ 12 years of age and have a genotype-confirmed mutation that is consistent with 1 of the 3 specified congenital neutropenias: GSD1b, G6PC3 deficiency, or GATA2 deficiency. Patients will be advised of the requirement of genetic screening in the discussion of the trial design and objectives. After signing the informed consent form (ICF), patients will undergo a blood test (or swab) to complete genetic screening for known severe congenital neutropenia, other chronic neutropenia disorders, and primary immunodeficiencies with neutropenia using targeted next-generation sequencing (NGS).

All eligible patients will be treated with mavorixafor at 400 mg by mouth (PO) (QD) in the morning for 14 days.

Patients with severe CIN or one of the selected congenital neutropenias and treated with prophylactic G-CSF at study initiation will not be allowed to have their G-CSF dose or regimen modified during the course of the study and must not have modified their G-CSF dose or regimen within 15 days (inclusive) before the start of study treatment. These patients will receive mavorixafor, 400 mg PO QD in ***addition*** to their standard G-CSF regimen for 14 days.

Baseline assessments at Day -1 for eligible patients will occur during a 6-hour hospitalization before the initiation of study drug and will consist of blood sampling to monitor ANC and ALC levels at the following times: 0, 30, 60, and 90 minutes (± 5 minutes each), and 2, 3, 4, and 6 hours (± 15 minutes each). These baseline ANC and ALC values will be averaged and will be thereafter referred to as baseline ANC and ALC. In addition, patients will have an ECG performed at time 0 and 4 hours later.

The administration of the first dose of 400 mg of mavorixafor will occur on Day 1. An ECG will be performed 4 hours post-dose. Blood sampling (PD) to monitor ANC and ALC levels and PK sampling will be performed on Day 1 at the following times: 0 (pre-dose and up to 15 minutes prior), 30, 60, and 90 minutes (± 5 minutes each), and 2, 3, 4, and 6 hours (± 15 minutes each) post-dose.

On Day 8, blood sampling (PK/PD) will be performed at the following times: 0 (pre-dose and up to 15 minutes prior), 30, 60, and 90 minutes (± 5 minutes each), and 2, 3, 4, and 6 hours (± 15 minutes each) post-dose. Hematology complete blood count and differential will additionally be performed for safety evaluation.

On Day 14 (end of treatment, or EOT), patients will receive their final dose of mavorixafor 400 mg and final blood sampling (PK/PD) at the following times: 0 (pre-dose and up to 15 minutes prior), 30, 60, and 90 minutes (± 5 minutes each), and 2, 3, 4, and 6 hours (± 15 minutes each) post-dose.

At the EOT, patients will continue their baseline G-CSF regimen at 100% of the original weekly dose.

### Patients with Selected Congenital Neutropenia/Not Treated with Prophylactic G-CSF

Patients with one of the selected congenital neutropenias who have not been treated with prophylactic G-CSF within 30 days of the start of the study, will receive mavorixafor alone, 400 mg PO QD, for 14 days.

Baseline assessments at Day -1 for eligible patients will occur during a 6-hour hospitalization before the initiation of study drug and will consist of blood sampling to monitor ANC levels at the following times: 0, 30, 60, and 90 minutes (± 5 minutes each) and 2, 3, 4, and 6 hours (± 15 minutes each). These baseline ANC and ALC values will be averaged and will be thereafter referred to as baseline ANC and ALC. In addition, patients will have an ECG performed at time 0 and 4 hours later.

The administration of the first dose of 400 mg of mavorixafor will occur on Day 1. An ECG will be performed 4-hours post-dose. Blood sampling (PD) to monitor ANC levels and PK sampling will be performed on Day 1 at the following times: 0 (pre-dose and up to 15 minutes prior), 30, 60, and 90 minutes (± 5 minutes each), and 2, 3, 4, and 6 hours (± 15 minutes each) post-dose.

On Day 8, blood sampling (PK/PD) will be performed at the following times: 0 (pre-dose and up to 15 minutes prior), 30, 60, and 90 minutes (± 5 minutes each), and 2, 3, 4, and 6 hours (± 15 minutes each) post-dose. Hematology complete blood count and differential will additionally be performed for safety evaluation.

On Day 14 (EOT), patients will receive their final dose of mavorixafor 400 mg and final blood sampling (PK/PD) at the following times: 0 (pre-dose and up to 15 minutes prior), 30, 60, and 90 minutes (± 5 minutes each), and 2, 3, 4, and 6 hours (± 15 minutes each) post-dose.

In all patients, in the event of an infection, patients may receive any standard-of-care antibiotic and/or procedure (i.e., drainage).

Patients will be monitored for safety and compliance throughout the study.

If on day 8 the neutrophil count is ≥30,000 cells/µL at any time point, the patient will discontinue mavorixafor. This will be considered a significant adverse effect (SAE) and the event would be followed until the outcome is known.

If on day 8 the neutrophil count is between 20,000 cells/µL and <30,000 cells/µL, the investigator has the option of monitoring the neutrophil count on days 10 and 12: if the neutrophil count is >30,000 cells/µL, the patient will discontinue mavorixafor. This will be considered an SAE and the event would be followed until the outcome is known.

All patients will attend an End of Study (EOS) visit at 30 days (± 5 days) posttreatment.

A study schema is presented in **FIG. 3****.** All assessments are to be conducted as described.

### Assessment of Efficacy

Absolute neutrophil count and ALC will be measured for the calculation of times above thresholds and AUCs. Patients are scheduled for blood sample collection at the following time points:
- Time 0 (pre-dose, up to 15 minutes prior), 30, 60, and 90 minutes (± 5 minutes) and 2, 3, 4, and 6 hours (± 15 minutes each) post-dose.
Absolute neutrophil count and ALC will be determined by standard methods. Whole blood samples will be sent to a central laboratory selected by the Sponsor.

All patients will attend an End of Study (EOS) visit at 30 days (± 5 days) posttreatment.

In order to assess the effects of mavorixafor, a detailed statistical analysis will be conducted. Data will be summarized and presented by disease group (CIN, congenital neutropenia). Tabulations will be produced for appropriate disposition, demographic, baseline characteristics, drug exposure, safety and tolerability, and efficacy parameters including ANC, ALC, AUC_{ANC}, and AUC_{ALC}. Summary statistics will be presented to analyze PK parameters and concentrations. Categorical variables will be summarized by frequency distributions (number and percentages of patients), and continuous variables will be summarized by descriptive statistics (mean, standard deviation, median, minimum, and maximum). No formal statistical testing will be performed.

### Reduction and/or Elimination of G-CSF

Presently, standard therapy for severe neutropenia, particularly in subjects with idiopathic neutropenia, i.e., of unknown cause, is treatment with granulocyte-colony stimulating factors ("G-CSFs") such as filgrastim, lenograstim or pegfilgrastim. However, treatment with G-CSF has several substantial drawbacks, including a high incidence of significant bone pain. Bone pain is estimated to occur in anywhere from 24% and [reported on filgrastim and pegfilgrastim labels, respectively] to as high as 66% for filgrastim [Ferguson (2015), Practical Pain Management, vol. 15 online at: practical painmanagement.com/treatments/pharmacological/non-opioids/antihistamine-g-csf-induced-bone-pain] and 59% (24% severe bone pain) for pegfilgrastim (Kirshner et al. (2012) J. Clin Oncol. 30:1974-79). G-CSF is also associated with flu-like symptoms. Further, a link between G-CSF and myeloid malignancies, such as myelodysplasia (MDS) or acute myeloid leukemia (AML) has been reported.

In some embodiments of the present invention, mavorixafor is used for treatment of patients with CIN at risk for infections.

It is anticipated by the inventors that administration of mavorixafor will permit reduction of the G-CSF for at least some patients. In some cases, this reduces the risk of G-CSF associated malignancy and myelofibrosis, and reduces G-CSF associated bone pain while maintaining protection from infection.

Patients will begin with a well-tolerated dose of oral, daily mavorixafor, for example, at 400 mg per day, to a patient who is presently receiving a full dose (1X) of G-CSF or peg-G-CSF. The patient will be monitored for ANC. If the patient's ANC is at or above 1000 cells/µL, then the clinician will consider reducing the patient's dose of G-CSF or peg-G-CSF by a factor of approximately 25%, (i.e., to 0.75X dose). If ANC remains at or above 1000 cells/µL, the clinician may consider (a) further reducing the patient's dose of G-CSF or peg-G-CSF; (b) revising (i.e., increasing or decreasing) the daily dosage of mavorixafor being administered; or both (a) and (b). ANC will continue to be monitored, with a goal of ANC of at least 500 cell/µL being maintained. As long as the patient's ANC remains above 500 cells/µL, the clinician may consider further reducing the patient's dose of G-CSF or peg-G-CSF with the goal of reducing bone pain or other adverse effects of G-CSF or peg-G-CSF, and will continue to monitor the ANC.

If the patient's measured ANC is found to be between 500 and 1000 cells/µL, the clinician may consider (a) further reducing the patient's dose of G-CSF or peg-G-CSF; (b) increasing the daily mavorixafor dosage; or both (a) and (b). ANC will continue to be monitored, with a goal of ANC of at least 500 cell/µL being maintained. As long as the patient's ANC remains above 500 cells/µL, the clinician may consider further reducing the patient's dose of G-CSF or peg-G-CSF with the goal of reducing bone pain or other adverse effects of G-CSF or peg-G-CSF, and will continue to monitor the ANC.

If the patient's measured ANC is found to be at or below 500 cells/µL, the clinician may consider (a) increasing the patient's dose of G-CSF or peg-G-CSF; (b) increasing the daily mavorixafor dosage; or both (a) and (b).

Long term studies will evaluate the ability to de-escalate G-CSF doses while maintaining ANC levels above 500 cells/µL. As long as the patient's ANC remains above 500 cells/µL, the clinician may consider further reducing the patient's dose of G-CSF or peg-G-CSF, and continue to monitor the ANC.

Other measures of clinical effectiveness or benefit may also be employed in order to determine the efficacy of a treatment regimen using mavorixafor or other CXCR4 inhibitor.
- Peripheral WBC counts (≥2 independent samples, obtained in the absence of signs or symptoms of acute infection, and when not having received G- or GM-CSF in the past 7 days) showing absolute neutrophil count <900/µL and/or absolute lymphocyte count <1,500/µL;
- Sustained increases in circulating neutrophils (e.g., ANC >600/µL; ANC >800/µL; ANC >1000/µL; or ANC >1,200/µL on at least 85% of assessments).
- Sustained increases in circulating lymphocytes (e.g., ALC >1000/µL; ALC >1,200/µL; or ALC >1,500/µL on at least 85% of assessments).
- Achieve pre-defined levels of protective antibody in response to at least 2 approved vaccines previously administered without achieving that level.
- 50% reduction in days of work or school missed due to infection
- Sustained increases in circulating neutrophils.

Not all endpoints are applicable to all patients with neutropenia. However, all patients exhibit at least one clinical and one laboratory metric.

Patients may preferably initiated on treatment orally with mavorixafor 25 mg once daily, 25 mg twice daily, or 50 mg once daily. Provision is made for dose reduction (which can be via increased interval; e.g., to every other day or twice weekly) in the event of toxicity or dose increase (e.g., to >50 mg once daily or higher daily dosage, such as 100 mg/day or 150 mg/day) in the event of an inadequate response.

An exemplary initial dosage is via mavorixafor 100 mg capsules, administered orally in the morning in a fasted state, with no food or drink (except water) after midnight and continuing until 2 hr post-dose. **In** twice daily dosage regimens, capsules are preferably administered orally twelve hours apart.

### Example 3: Clinical Treatment Regimens

### Dosing Regimen for Patients with Chronic Neutropenia or Congenital Neutropenia:

If the patient experiences adverse effects at any time, in particular a treatment-limiting toxicity, as defined by the National Cancer Institute Common Terminology Criteria for Adverse Events, version 4.03), provision is made for dose reduction (i.e., lower dosage and/or increased interval between administrations drug), or administration is halted. Additionally, the treating physician may use his or her professional judgment and discretion in determining the starting dose, and how best to titrate to the appropriate dose of mavorixafor for any individual patient.

Exemplary compositions of mavorixafor 25 mg, 100 mg, and 200 mg capsules that may be used in methods disclosed herein are shown in **Table 10A, 10B,** and **10C** below.

**Table 10A: Quantitative Composition of Exemplary Mavorixafor 25 mg Capsule**

| **Component** | **Reference to Standard** | **Function** | **Quantity (mg/capsule)** | **% w/w** |
|---|---|---|---|---|
| Mavorixafor | In House | Active Ingredient | 25.0 | 14.7 |
| Microcrystalline Cellulose | NF | Diluent | 132.7 | 78.1 |
| Croscarmellose Sodium | NF | Disintegrant | 10.2 | 6.0 |
| Sodium Stearyl Fumarate | NF | Lubricant | 1.7 | 1.0 |
| Colloidal Silicon Dioxide | USP | Flow Aid | 0.4 | 0.2 |
| **Sum Total** | | | **170** | **100.0** |
| Hard Gelatin Capsules, Size 1 | USP | Packaging | NA | NA |

**Table 10B: Composition of X4P-001 100 mg Capsules**

| **Component** | **Reference to Standard** | **Function** | **100 mg** | |
|---|---|---|---|---|
| | | | **Quantity (mg/capsule)** | **w/w** |
| X4P-001 composition | In House | Active substance | 100.0 | 37.6% |
| Dibasic Calcium Phosphate Dihydrate | USP/NF | Diluent | 84.3 | 31.7% |
| Microcrystalline Cellulose | NF/EP | Diluent | 60.9 | 22.9% |
| Croscarmellose Sodium | NF/EP | Disintegrant | 16.0 | 6.0% |
| Sodium Stearyl Fumarate | NF | Lubricant | 2.7 | 1.0% |
| Sodium Lauryl Sulfate | NF/EP | Wetting agent | 1.3 | 0.5% |
| Colloidal Silicon Dioxide | NF/EP | Flow Aid | 0.8 | 0.3% |
| **Sum** | | | **266.0** | **100%** |
| Hard gelatin capsules, Size 1 white/white. Qualitative composition: Gelatin and Titanium dioxide. | USP | Encapsulation | N/A | N/A |

**Table 10C: Composition of X4P-001 200 mg Capsules**

| | **200 mg** | |
|---|---|---|
| **Ingredients** | **Percent Per Capsule (%)** | **Theoretical Amount Per Capsule (mg)** |
| X4P-001 composition | 61.5 | 200.0 |
| Microcrystalline Cellulose, NF/EP (Avicel PH 101) or equivalent | 12.9 | 41.93 |
| Dibasic Calcium Phosphate Dihydrate, USP/NF | 17.8 | 57.85 |
| Croscarmellose Sodium, NF/EP (Ac-Di-Sol) | 6.0 | 19.50 |
| Sodium Lauryl Sulfate, NF / Ph. Eur. | 0.5 | 1.625 |
| Colloidal Silicone Dioxide, NF/ Ph. Eur. (Cab-O-Sil M-5P) | 0.3 | 0.9750 |
| Sodium Stearyl Fumarate, NF (Pruv) | 1.0 | 3.250 |
| **Total Capsule Fill** | **100** | **325.0** |

### Example 4: Assessments of Treatment Effect

### Circulating White Blood Cells

Whole blood samples are analyzed for CBC and absolute leukocyte differential counts by standard laboratory methods, including WBC counts, including absolute numbers of lymphocytes, neutrophils, and CD34+ cells. The number and percentage of patients achieving ANC >1,500/µL; ALC >900/µL. The absolute increase in blood neutrophil counts from pretreatment baseline for each subject, including at the maximum observed in the hours post-dosing; and the maximum observed pre-dose on stable drug administration regimen. These results are compared with data from healthy adults administered X4P-001.

Peripheral Blood Mononuclear Cells (PBMC) subpopulations by flow cytometry are shown below in Table 11.

**Table 11: Candidate Subsets of Circulating Lymphocytes and Monocytes**

| | | |
|---|---|---|
| CD4+ T cells | CD3- CD56+ (NK cells) | CD34+ (stem cells) |
| CD4+ CD45RA+ (naive T cells) | CD19+ (B cells) | CD49f+ (stem cells) |
| | CD19+ CD27- IgM+ (transitional B cells) | CD90+ (stem cells) |
| CD4+ CD45RA-(memory T cells) | | |
| | CD14+ (monocytes) | |
| CD8+ T cells | CD14+ CD16- (classical | |
| CD8+ CD45RA+ (naive T cells) | monocytes) | |
| | CD14+ CD16+ (inflammatory monocytes) | |
| CD8+ CD45RA-(memory T cells) | | |

### Pharmacokinetic Assessments

If desired, pharmacokinetic assessment of blood samples for plasma levels of X4P-001 may be conducted. Blood samples are collected as scheduled. Samples are analyzed for X4P-001 concentration using reversed-phase high performance liquid chromatography (RP-HPLC) with MS/MS detection. The validated range of this bioanalytic method is 30 to 3,000 ng/mL in plasma.

**Pharmacokinetics (PK) and Pharmacodynamics (PD).** In order to evaluate the pharmacokinetic properties of therapy with X4P-001, levels of X4P-001, PK samples may be obtained on all patients in Part A as follows:
- Day 1: pre-dose; post-dose at 30, 60, 90 min (each ±10%) and 2, 3, 4 hr (each ±15 min)
- Day 8 visit: pre-dose; post-dose at 30, 60, 90 min (each ±10%) and 2, 3, 4, 6 hr (each ±15 min)
- Day 14 visit: pre-dose; post-dose at 30, 60, 90 min (each ±10%) and 2, 3, 4, 6 hr (each ±15 min)

Visits are scheduled for early in the day and patients are instructed to arrive at the clinic fasting and having not taken their morning dose of X4P-001.

PK are analyzed by patient and dosage regimen over the preceding week using descriptive statistics for AUC, Cmax, and Cmin.

**PD samples** are collected on Day 1, Day 8 and Day 14 visit concurrent with scheduled PK samples (see above) for:
- Total white blood cell (WBC) counts, ANC and ALC.
- Assessments may include samples analyzed by flow cytometry for subpopulations of PBMCs.

Of course, the treating physician may apply his or her professional judgment and discretion and any established standards of care, what parameters of assessment (e.g., the desired levels of ANC and ALC) should be used in determining the treatment regimen for any individual patient.

### References

Baggiolini; 1998. Chemokines and leukocyte traffic. Nature. 392: 565-568.
Bainton et al. (1971) The development of neutrophilic polymorphonuclear leukocytes in human bone marrow. J Exp Med. 134:907-34.
Balabanian, et al. 2012. Proper desensitization of CXCR4 is required for lymphocyte development and peripherial compartmentalization in mice. Blood. 119: 5722-5730.
Banka et al. (2011) Variability of bone marrow morphology in G6PC3 mutations: is there a genotype-phenotype correlation or age-dependent relationship? Am J Hematol. 86:235-7.
Banka and Newman (2013) A clinical and molecular review of ubiquitous glucose-6-phosphatase deficiency caused by G6PC3 mutations. Orphanet J Rare Dis. 8:84.
Banka (2015) G6PC3 deficiency: synonym: ubiquitous glucose-6-phosphatase deficiency. GeneReviews [internet] 2015; Adam et al., editors. Seattle WA: University of Washington, Seattle; 1993-2019.
Broxmeyer et al. (2005) Rapid mobilization of murine and human hematopoietic stem and progenitor cells with AMD3100, a CXCR4 antagonist. J Exp Med 201:1307-18.
Boztug et al. (2009) A syndrome with congenital neutropenia and mutations in G6PC3. N Engl J Med.; 360:32-43.
Cao, et al. (2008) Effect of Low-Dose Ritonavir on the Pharmacokinetics of the CXCR4 Antagonist AMD070 in Healthy Volunteers. Antimicrob Agents Chemother. 52:1630-1634.
Chou and Mansfield (2003) Glucose-6-phosphate transporter: the key to glycogen storage disease type Ib. In: Broer and Wagner, editors. Membrane Transporter Diseases. New York: Springer; 191-205.
Chou et al. (2010) Neutropenia in type 1b glycogen storage disease. Curr Opin Hematol. 17:36-42.
Crispino and Horwitz (2017) GATA factor mutations in hematologic disease. Blood 129:2103-10.
Crump, et al. (1997) Solution structure and basis for functional activity of stromal cell derived factor-1; dissociation of CXCR4 activation from binding and inhibition of HIV-1. EMBO J. 16:6996-7007
Dale et al. (1993) A randomized controlled phase III trial of recombinant human granulocyte colony-stimulating factor (filgrastim) for treatment of severe chronic neutropenia. Blood 81:2496-502.
Dale, et al. (2006) The Severe Chronic Neutropenia International Registry: 10-Year Follow-up Report. Support Cancer Ther. 3:220-31.
Dale and Bolyard (2017) An update on the diagnosis and treatment of chronic idiopathic neutropenia. Curr Opin Hematol. 24:46-53.
Dale et al. (2019) Neutropenia in glycogen storage disease !b: outcomes for patients treated with granulocyte colony-stimulating factor. Curr Opin Hematol. 26:16-21.
Doranz (1997). Chemokine receptors as fusion cofactors for human immunodeficiency virus type 1 (HIV-1). Immunol Res. 16:15-28.
Dresch et al. (1975) Kinetic studies of 51Cr and DF32P labelled granulocytes. Br J Haematol. 29:67-80.
Eash et al. (2009) CXCR4 is a key regulator of neutrophil release from the bone marrow under basal and stress granulopoiesis conditions. Blood. 113:4711-19.
FDA Drug development and drug interactions: table of substrates, inhibitors and inducers. Updated 14 November 2017. Available at fda.gov/drugs/drug-interactions-labeling/drug-development-and-drug-interactions-table-substrates-inhibitors-and-inducers. Accessed 22 June 2019.
Galsky, et al. (2014). A Phase I Trial of LY2510924, a CXCR4 Peptide Antagonist, in Patients with Advanced Cancer. Clin Cancer Res. doi:10.1158/1078-0432.CCR-13-2686.
Goldman et al. (2016) Hyperglycemia associated with targeted oncologic treatment: mechanisms and management. Oncologist. 21:1326-36.
Greenberg et al. (1980) The chronic idiopathic neutropenia syndrome: correlation of clinical features with in vitro parameters of granulocytopoiesis. Blood. 55:915-921.
Hayee, et al. (2011) G6PC3 mutations are associated with a major defect of glycosylation: a novel mechanism for neutrophil dysfunction. Glycobiology, 21:914-24.
Hendrix, et al. 2004. Safety, Pharmacokinetics, and Antiviral Activity of AMD3100, a Selective CXCR4 Receptor Inhibitor, in HIV-1 Infection. J Acquir Immune Defic Syndr. 37:1253-1262.
Hickstein (2018) HSCT for GATA2 deficiency across the pond. Blood. 131:1272-74. Hsu et al. (2015) GATA2 deficiency. Curr Opin Allergy Clin Immunol. 15:104-9.
Kawai, et al.;. WHIM syndrome myelokathexis reproduced in the NOD/SCID mouse xenotransplant model engrafted with healthy human stem cells transduced with C terminustruncated CXCR4. Blood. 2007; 109:78-84. Epub 2006 Aug 31.
Kim et al. (2006) G-CSF down-regulation of CXCR3 expression identified as a mechanism for mobilization of myeloid cells. Blood. 108:812-20.
Kim et al. (2008) Neutrophil stress and apoptosis underlie myeloid dysfunction in glycogen storage disease type Ib. Blood. 111:5704-11.
Kyle and Linman. (1968) Chronic idiopathic neutropenia. A newly recognized entity? N Engl J Med. 279:1015-19.
Lagane, et al. (2008) CXCR4 dimerization and beta-arrestin-mediated signaling account for the enhanced chemotaxis to CXCL12 in WHIM syndrome. Blood. 112:34-44.
Lapidot and Petit (2002) Current understanding of stem cell mobilization: the roles of chemokines, proteolytic enzymes, adhesion molecules, cytokines, and stromal cells. Exp Hematol. 30:973-981.
Levesque et al. (2003) Disruption of the CXCR4/CXCR12 chemotactic interaction during hematopoietic stem cell mobilization induced by G-CSF or cyclophosphamide. J Clin Invest. 111:187-96.
Liles et al. (2003) Mobilization of hematopoietic progenitor cells in healthy volunteers by AMD3100, a CXCR4 antagonist. Blood. 102:2728-30.
Link (2005) Neutrophil homeostasis: a new role for stromal cell-derived factor-1. Immunol Res.32:169-78.
Lord et al. (1991) Myeloid cell kinetics in mice treated with recombinant interleukin-3, granulocyte colony-stimulating factor (GSF), or granulocyte-macrophage CSF in vivo. Blood. 77:2154-9.
Ma et al. (1999) The chemokine receptor CXCR4 is required for retention of B lineage and granulocytic precursors in the bone marrow microenvironment. Immunity. 10:463-471.
Maciejewski-Duval et al. (2016) Altered chemotactic response to CXCL12 in patients carrying GATA2 mutations. J Leukoc Bio. 99:1065-76.
Martin et al. (2014) Births in the United States, 2013. National Center for Health Statistics Data Brief. No. 175: December 2014.
McCormick et al. (2009) Impaired recruitment of Grk6 and beta-Arrestin 2 causes delayed internalization and desensitization of a WHIM syndrome-associated CXCR4 mutant receptor. PLoS One. 4:e8102.
   a) McDermott et al. (2011) The CXCR4 antagonist plerixafor corrects panleukopenia in patients with WHIM syndrome. Blood.118:4957-62.
   b) McDermott et al (2014) A phase 1 clinical trial of long-term, low-dose treatment of WHIM syndrome with the CXCR4 antagonist plerixafor. Blood. 123:2308-16.
   c) McDermott et al. (2010) Severe congenital neutropenia resulting from G6PC3 deficiency with increased neutrophil CXCR4 expression and myelokathexis. Blood 116:2793-802.
Mosi, et al. (2012) The molecular pharmacology of AMD11070: An orally bioavailable CXCR4 HIV entry inhibitor. Biochem Pharmacology. 83:472-479.
Moyle, et al. (2009) Proof of Activity with AMD11070, an Orally Bioavailable Inhibitor of CXCR4-Tropic HIV Type 1. Clin Infect Dis. 48:798-805.
Nyunt, et al. (2008) Pharmacokinetic Effect of AMD070, an Oral CXCR4 Antagonist, on CYP3A4 and CYP2D6 Substrates Midazolam and Dextromethorphan in Healthy Volunteers. J Acquir Immune Defic Syndr. 47:559-565.
Parker. (2001) Glucose-6-phosphate translocase as a target for the design of antidiabetic agents. Drugs Fut. 26:687.
Pillay et al. (2010) In vivo labeling with 2H2O reveals a human neutrophil lifespan of 5.4 days. Blood. 116:625-7.
Roe et al. (2002) Inflammatory bowel disease in glycogen storage disease type 1b. J Pediatr 109:55-9.
Semerad et al. (2002) G-CSF is an essential regulator of neutrophil trafficking from the bone marrow to the blood. Immunity. 17:413-23.
Sicre de Fontbrune et al. (2015) Severe chronic primary neutropenia in adults: report on a series of 108 patients. Blood. 126:1643-50.
Stone, et al. (2007) Multiple-Dose Escalation Study of the Safety, Pharmacokinetics, and Biologic Activity of Oral AMD070, a Selective CXCR4 Receptor Inhibitor, in Human Subjects. Antimicrob Agents Chemother. 51:2351-2358.
Suratt et al. (2004) Role of the CXCR4/SDF-1 chemokine axis in circulating neutrophil homeostasis. Blood. 104:565-71.
Ueno et al. (1986) Impaired monocyte function in glycogen storage disease type 1d. Eur J Pediatr. 145:312-14.
Veiga-da-Cunha et al. (2019) Failure to eliminate a phosphorylated glucose analog leads to neutropenia in patients with G6PT and G6PC#3 deficiencies. Proc Natl Acad Sci USA. 116:1241-50.
Vinh et al. (2010) Autosomal dominant and sporadic monocytopenia with susceptibility to mycobacteria, fungi, papillomaviruses, and myelodysplasia. Blood. 115:1519-29.
Visser et al. (2000) Neutropenia, neutrophil dysfunction, and inflammatory bowel disease in glycogen storage disease type Ib: results of the European Study on Glycogen Storage Disease type I. J Pediatr. 137:187-91.
Visser et al. (2006) Granulocyte colony-stimulating factor in glycogen storage disease type 1b. Results of the European study on glycogen storage disease type 1. Eur J Pediatr. 161(Suppl 1):S83-7.
Ward and Dale (2009) Genetic and molecular diagnosis of severe congenital neutropenia. Curr Opin Hematol. 16:9-13.
Wong (2008) Comparison of the potential multiple binding modes of bicyclam, monocylam, and noncyclam small molecule CXC chemokine receptor 4 inhibitors. Mol Pharmacol. 74:1485-1495.
Zlotnik and Yoshie (2000) Chemokines: a new classification system and their role in immunity. Immunity. 12:121-127.

### INTERNET References:

George Diaz, Virginia Gulino. Whim syndrome. Orphanet Encyclopedia. June 2004; orpha.net/data/patho/GB/uk-Whim.pdf
NORD (National Organization for Rare Diseases) 2015: rarediseases.org/rarediseases/whimsyndrome
Office of Rare Diseases: rarediseases.info.nih.gov/gard/9297/whimsyndrome/resources/1
Orphanet, WHIM Syndrome:
orpha.net/consor/www/cgibin/OC_Exp.php?lng=EN&Expert=51636 Last Update October 2014.
US Census Bureau: census.gov/quickfacts/table/PST045214/00 Last Revised June 2015.

## Claims

1. Mavorixafor or a pharmaceutically acceptable salt or composition thereof for use in a method for treating chronic neutropenia, cyclic neutropenia, or congenital neutropenia in a patient in need thereof, wherein the patient does not have a gain-of-function mutation in the *CXCR4* gene and does not have a genetic abnormality associated with WHIM syndrome, said use comprising administering to the patient an effective amount of mavorixafor or a pharmaceutically acceptable salt or composition thereof,
wherein G-CSF is co-administered to the patient, wherein the dosage of G-CSF that is administered to the patient is reduced by at least 25% relative to the patient's previous dose before beginning treatment with mavorixafor or a pharmaceutically acceptable salt or composition thereof; optionally:
wherein the patient has severe chronic neutropenia (SCN), or chronic idiopathic neutropenia (CIN), or autoimmune neutropenia (AIN); and/or
wherein the patient has an absolute neutrophil count (ANC) less than 1500 cells/µL and/or an absolute lymphocyte count (ALC) less than 1000 cells/µL; or
wherein the patient has an absolute neutrophil count (ANC) less than 500 cells/µL and/or an absolute lymphocyte count (ALC) less than 650 cells/µL.

2. Mavorixafor or a pharmaceutically acceptable salt or composition thereof for use of claim 1, wherein the patient is experiencing adverse effects attributed to the therapy with G-CSF.

3. Mavorixafor or a pharmaceutically acceptable salt or composition thereof for use of claim 1 or claim 2, wherein the patient has a genetic abnormality selected from GSD1b, G6PC3 deficiency, GATA2 deficiency, a genetically-defined condition with or without myeloid maturation arrest at the myelocyte/promyelocyte stage, suspected aplastic anemia, B-cell immunodeficiency, juvenile myelodysplastic syndrome (MDS), chronic myelomonocytic leukemia, a severe Epstein-Barr virus infection or Epstein-Barr-associated cancers, B-cell acute lymphoblastic leukemia, or unexplained bone marrow failure.

4. Mavorixafor or a pharmaceutically acceptable salt or composition thereof for use of any one of claims 1 to 3, wherein mavorixafor or a pharmaceutically acceptable salt thereof is administered at a dose of from about 100 mg/day to about 600 mg/day; or: wherein mavorixafor or a pharmaceutically acceptable salt thereof is administered at a dose of about 400 mg/day.

5. Mavorixafor or a pharmaceutically acceptable salt or composition thereof for use in a method for reducing the risk of an infection in a patient with neutropenia, wherein the patient with neutropenia does not have a gain-of-function mutation in the *CXCR4* gene and does not have a genetic abnormality associated with WHIM syndrome, said use comprising administering to the patient an effective amount of mavorixafor or a pharmaceutically acceptable salt or composition thereof,
wherein G-CSF is co-administered to the patient, wherein the dosage of G-CSF that is administered to the patient is reduced by at least 25% relative to the patient's previous dose before beginning treatment with mavorixafor or a pharmaceutically acceptable salt or composition thereof; optionally:
wherein the patient has severe chronic neutropenia (SCN), chronic idiopathic neutropenia (CIN), or autoimmune neutropenia (AIN); and/or
wherein the patient has an absolute neutrophil count less than 500 cells/µL.

6. Mavorixafor or a pharmaceutically acceptable salt or composition thereof for use of claim 5, wherein the patient is experiencing adverse effects attributed to the therapy with G-CSF.

7. Mavorixafor or a pharmaceutically acceptable salt or composition thereof for use of claim 5 or claim 6, wherein the patient has an elevated risk of an infection selected from respiratory tract infections, otitis media, stomatitis, urinary tract infections, pyelonephritis, skin abscesses, cellulitis, and sepsis.

8. Mavorixafor or a pharmaceutically acceptable salt or composition thereof for use of any one of claims 5 to 7, wherein mavorixafor or a pharmaceutically acceptable salt thereof is administered at a dose of from about 100 mg/day to about 600 mg/day; or: wherein mavorixafor or a pharmaceutically acceptable salt thereof is administered at a dose of about 400 mg/day.

9. Mavorixafor or a pharmaceutically acceptable salt or composition thereof for use in a method for reducing the dosage of granulocyte colony-stimulating factor (G-CSF) for treating severe chronic neutropenia (SCN) in a patient in need thereof, wherein the patient does not have a gain-of-function mutation in the *CXCR4* gene and does not have a genetic abnormality associated with WHIM syndrome, said use comprising administering to the patient an effective amount of mavorixafor or a pharmaceutically acceptable salt or composition thereof; wherein the dosage of G-CSF that is administered to the patient is reduced by at least about 25% relative to the patient's previous dose before beginning treatment with mavorixafor or a pharmaceutically acceptable salt or composition thereof;
optionally:
wherein the dosage of G-CSF that is administered to the patient is reduced by at least about 50% relative to the patient's previous dose before beginning treatment with mavorixafor or a pharmaceutically acceptable salt or composition thereof.

10. Mavorixafor or a pharmaceutically acceptable salt or composition thereof for use of claim 9, wherein mavorixafor or a pharmaceutically acceptable salt thereof is administered in a dose of from about 100 mg/day to about 600 mg/day; and/or: wherein mavorixafor or a pharmaceutically acceptable salt thereof is administered at a dose of about 400 mg/day.

11. Mavorixafor or a pharmaceutically acceptable salt or composition thereof for use of claim 9 or claim 10, wherein (I) the patient has a congenital neutropenia condition; optionally wherein the congenital neutropenia condition is selected from the group consisting of: GSD1b due to mutations in *SLC37A4*; G6PC3 deficiency due to mutations in *G6PC3,* and GATA2 deficiency due to mutations in *GATA2*; or:
wherein (II) the patient has a chronic idiopathic neutropenia (CIN) condition.

12. Mavorixafor or a pharmaceutically acceptable salt or composition thereof for use of any one of claims 9 to 11, wherein the patient exhibits an absolute neutrophil count (ANC) less than 500 cells/µL prior to treatment; or:
wherein the patient exhibits an absolute neutrophil count (ANC) less than 600 cells/µL prior to treatment.

13. Mavorixafor or a pharmaceutically acceptable salt or composition thereof for use of any one of claims 9 to 12, wherein the patient is experiencing adverse effects attributed to the G-CSF therapy; optionally wherein the method provides a decrease in the incidence of bone pain in the patient, a decrease in the incidence of flu-like symptoms in the patient, or a decrease in the incidence of a myeloid malignancy in the patient.

14. Mavorixafor or a pharmaceutically acceptable salt or composition thereof for use of any one of claims 1 to 13, wherein the use results in an increase in absolute neutrophil count (ANC) to at least 800 cells/µL; or cell counts of WBC, neutrophils, and/or lymphocytes increase to approximately 2.0 x baseline.

## Patentansprüche

1. Mavorixafor, oder ein pharmazeutisch verträgliches Salz oder eine Zubereitung davon, zur Verwendung in einem Verfahren zur Behandlung von chronischer Neutropenie, zyklischer Neutropenie oder angeborener Neutropenie bei einem Patienten, der einer solchen Behandlung bedarf, wobei der Patient keine Funktionsgewinnmutation ("gain of function mutation") im *CXCR4*-Gen aufweist, und keine mit dem WHIM-Syndrom assoziierte genetische Anomalie aufweist, wobei die Verwendung die Verabreichung einer wirksamen Menge von Mavorixafor oder eines pharmazeutisch verträglichen Salzes oder einer Zubereitung davon an den Patienten umfasst,
wobei dem Patienten G-CSF gleichzeitig verabreicht wird, wobei die dem Patienten verabreichte G-CSF-Dosis im Vergleich zur vorherigen Dosis des Patienten vor Beginn der Behandlung mit Mavorixafor oder einem pharmazeutisch verträglichen Salz oder einer Zubereitung davon um mindestens 25 % reduziert ist; optional:
wobei der Patient an schwerer chronischer Neutropenie (SCN) oder chronischer idiopathischer Neutropenie (CIN) oder autoimmuner Neutropenie (AIN) leidet; und/oder
wobei der Patient eine absolute Neutrophilenzahl (ANC) von weniger als 1500 Zellen/µL und/oder eine absolute Lymphozytenzahl (ALC) von weniger als 1000 Zellen/µL aufweist; oder
wobei der Patient eine absolute Neutrophilenzahl (ANC) von weniger als 500 Zellen/µL und/oder eine absolute Lymphozytenzahl (ALC) von weniger als 650 Zellen/µL aufweist.

2. Mavorixafor, oder ein pharmazeutisch verträgliches Salz oder eine Zubereitung davon, zur Verwendung nach Anspruch 1, wobei der Patient unter Nebenwirkungen leidet, die auf die Therapie mit G-CSF zurückzuführen sind.

3. Mavorixafor, oder ein pharmazeutisch verträgliches Salz oder eine Zubereitung davon, zur Verwendung nach Anspruch 1 oder Anspruch 2, wobei der Patient eine genetische Anomalie aufweist, ausgewählt aus GSD1b-, G6PC3-Mangel, GATA2-Mangel, einer genetisch definierten Erkrankung mit oder ohne myeloischen Reifungsstillstand im Myelozyten-/Promyelozytenstadium, Verdacht auf aplastische Anämie, B-Zell-Immundefekt, jugendliches myelodysplastisches Syndrom (MDS), chronische myelomonozytäre Leukämie, schwere Epstein-Barr-Virus-Infektion oder Epstein-Barr-assoziierte Krebserkrankungen, akute B-Zell-Lymphoblasten-leukämie oder ungeklärtes Knochenmarkversagen.

4. Mavorixafor, oder ein pharmazeutisch verträgliches Salz oder eine Zusammensetzung davon, zur Verwendung gemäß einem der Ansprüche 1 bis 3, wobei Mavorixafor oder ein pharmazeutisch verträgliches Salz davon in einer Dosis von etwa 100 mg/Tag bis etwa 600 mg/Tag verabreicht wird; oder wobei Mavorixafor oder ein pharmazeutisch verträgliches Salz davon in einer Dosis von etwa 400 mg/Tag verabreicht wird.

5. Mavorixafor, oder ein pharmazeutisch verträgliches Salz oder eine Zubereitung davon, zur Verwendung in einem Verfahren zur Verringerung des Infektionsrisikos bei einem Patienten mit Neutropenie, wobei der Patient mit Neutropenie keine Funktionsgewinnmutation im *CXCR4*-Gen aufweist und keine mit dem WHIM-Syndrom assoziierte genetische Anomalie aufweist, wobei die Verwendung die Verabreichung einer wirksamen Menge an Mavorixafor, oder eines pharmazeutisch verträglichen Salzes oder einer pharmazeutisch verträglichen Zusammensetzung davon, an den Patienten umfasst,
wobei dem Patienten G-CSF gleichzeitig verabreicht wird, wobei die dem Patienten verabreichte G-CSF-Dosis im Vergleich zur vorherigen Dosis des Patienten vor Beginn der Behandlung mit Mavorixafor, oder einem pharmazeutisch verträglichen Salz oder einer pharmazeutisch verträglichen Zusammensetzung davon, um mindestens 25 % reduziert ist; optional:
wobei der Patient an schwerer chronischer Neutropenie (SCN), chronischer idiopathischer Neutropenie (CIN) oder autoimmuner Neutropenie (AIN) leidet; und/oder
wobei der Patient eine absolute Neutrophilenzahl von weniger als 500 Zellen/µL aufweist.

6. Mavorixafor, oder ein pharmazeutisch verträgliches Salz oder eine Zubereitung davon, zur Verwendung gemäß Anspruch 5, wobei der Patient unter Nebenwirkungen leidet, die auf die Therapie mit G-CSF zurückzuführen sind.

7. Mavorixafor, oder ein pharmazeutisch verträgliches Salz oder eine Zubereitung davon, zur Verwendung nach Anspruch 5 oder Anspruch 6, wobei der Patient ein erhöhtes Risiko für eine Infektion aufweist, die aus Atemwegsinfektionen, Mittelohrentzündung, Stomatitis, Harnwegsinfektionen, Pyelonephritis, Hautabszessen, Zellulitis und Sepsis ausgewählt ist.

8. Mavorixafor oder ein pharmazeutisch verträgliches Salz oder eine Zubereitung davon zur Verwendung gemäß einem der Ansprüche 5 bis 7, wobei Mavorixafor oder ein pharmazeutisch verträgliches Salz davon in einer Dosis von etwa 100 mg/Tag bis etwa 600 mg/Tag verabreicht wird; oder wobei Mavorixafor, oder ein pharmazeutisch verträgliches Salz davon, in einer Dosis von etwa 400 mg/Tag verabreicht wird.

9. Mavorixafor, oder ein pharmazeutisch verträgliches Salz oder eine Zubereitung davon, zur Verwendung in einem Verfahren zur Reduzierung der Dosis von Granulozyten-Kolonie-stimulierendem Faktor (G-CSF) zur Behandlung einer schweren chronischen Neutropenie (SCN) bei einem Patienten, der dies benötigt, wobei der Patient keine Funktionsgewinnmutation im *CXCR4*-Gen aufweist und keine mit dem WHIM-Syndrom assoziierte genetische Anomalie aufweist, wobei die Verwendung die Verabreichung einer wirksamen Menge von Mavorixafor, oder eines pharmazeutisch verträglichen Salzes oder einer Zubereitung davon, an den Patienten umfasst; wobei die dem Patienten verabreichte G-CSF-Dosis um mindestens etwa 25 % gegenüber der vorherigen Dosis des Patienten vor Beginn der Behandlung mit Mavorixafor, oder einem pharmazeutisch verträglichen Salz oder einer Zubereitung davon, reduziert wird;
optional:
wobei die dem Patienten verabreichte G-CSF-Dosis im Vergleich zur vorherigen Dosis des Patienten vor Beginn der Behandlung mit Mavorixafor oder einem pharmazeutisch verträglichen Salz oder einer Zubereitung davon um mindestens etwa 50 % reduziert wird.

10. Mavorixafor, oder ein pharmazeutisch verträgliches Salz oder eine Zubereitung davon, zur Verwendung nach Anspruch 9, wobei Mavorixafor, oder ein pharmazeutisch verträgliches Salz davon, in einer Dosis von etwa 100 mg/Tag bis etwa 600 mg/Tag verabreicht wird; und/oder wobei Mavorixafor, oder ein pharmazeutisch verträgliches Salz davon, in einer Dosis von etwa 400 mg/Tag verabreicht wird.

11. Mavorixafor, oder ein pharmazeutisch verträgliches Salz oder eine Zubereitung davon, zur Verwendung nach Anspruch 9 oder Anspruch 10, wobei (I) der Patient an einer angeborenen Neutropenie leidet; gegebenenfalls wobei die angeborene Neutropenie aus der Gruppe ausgewählt ist, bestehend aus: GSD1b aufgrund von Mutationen in *SLC37A4*; G6PC3-Mangel aufgrund von Mutationen in *G6PC3* und GATA2-Mangel aufgrund von Mutationen in *GATA2*; oder:
wobei (II) der Patient an einer chronischen idiopathischen Neutropenie (CIN) leidet.

12. Mavorixafor, oder ein pharmazeutisch verträgliches Salz oder eine Zubereitung davon, zur Verwendung gemäß einem der Ansprüche 9 bis 11, wobei der Patient vor der Behandlung eine absolute Neutrophilenzahl (ANC) von weniger als 500 Zellen/µL aufweist; oder:
wobei der Patient vor der Behandlung eine absolute Neutrophilenzahl (ANC) von weniger als 600 Zellen/µL aufweist.

13. Mavorixafor, oder ein pharmazeutisch verträgliches Salz oder eine Zubereitung davon, zur Verwendung gemäß einem der Ansprüche 9 bis 12, wobei der Patient Nebenwirkungen erfährt, die auf die G-CSF-Therapie zurückzuführen sind; optional, wobei das Verfahren eine Verringerung der Häufigkeit von Knochenschmerzen beim Patienten, eine Verringerung der Häufigkeit von grippeähnlichen Symptomen beim Patienten oder eine Verringerung der Häufigkeit einer myeloischen Malignität beim Patienten bewirkt.

14. Mavorixafor, oder ein pharmazeutisch verträgliches Salz oder eine Zusammensetzung davon, zur Verwendung gemäß einem der Ansprüche 1 bis 13, wobei die Verwendung zu einem Anstieg der absoluten Neutrophilenzahl (ANC) auf mindestens 800 Zellen/µL führt; oder die Zellzahlen der weißen Blutkörperchen (WBC), Neutrophilen und/oder Lymphozyten auf etwa das 2,0-Fache des Ausgangswerts ansteigen.

## Revendications

1. Mavorixafor, ou un sel ou une composition pharmaceutiquement acceptable de celui-ci, pour utilisation dans un procédé de traitement de la neutropénie chronique, de la neutropénie cyclique ou de la neutropénie congénitale chez un patient qui en a besoin, dans lequel le patient ne présente pas de mutation à gain de fonction dans le gène *CXCR4* et ne présente pas d'anomalie génétique associée au syndrome WHIM, ladite utilisation consistant à administrer au patient une quantité efficace de mavorixafor ou d'un sel ou d'une composition pharmaceutiquement acceptable de celui-ci,
dans lequel du G-CSF est co-administré au patient, la dose de G-CSF administrée au patient étant réduite d'au moins 25 % par rapport à la dose précédente du patient avant le début du traitement par le mavorixafor ou un sel ou une composition pharmaceutiquement acceptable de celui-ci ; éventuellement :
dans lequel le patient présente une neutropénie chronique sévère (SCN), ou une neutropénie chronique idiopathique (CIN), ou une neutropénie auto-immune (AIN) ; et/ou
dans lequel le patient présente un nombre absolu de neutrophiles (ANC) inférieur à 1 500 cellules/µL et/ou un nombre absolu de lymphocytes (ALC) inférieur à 1 000 cellules/µL ; ou
dans lequel le patient présente un nombre absolu de neutrophiles (ANC) inférieur à 500 cellules/µL et/ou un nombre absolu de lymphocytes (ALC) inférieur à 650 cellules/µL.

2. Mavorixafor, ou un sel ou une composition pharmaceutiquement acceptable de celui-ci, pour utilisation selon la revendication 1, dans lequel le patient présente des effets indésirables attribués à la thérapie avec le G-CSF.

3. Mavorixafor, ou un sel ou une composition pharmaceutiquement acceptable de celui-ci, pour utilisation selon la revendication 1 ou la revendication 2, dans lequel le patient présente une anomalie génétique choisie parmi le déficit en GSD1b, le déficit en G6PC3, le déficit en GATA2, une affection génétiquement définie avec ou sans arrêt de la maturation myéloïde au stade myélocyte/promyélocyte, une anémie aplasique présumée, une immunodéficience à cellules B, un syndrome myélodysplasique juvénile (MDS), une leucémie myélomonocytaire chronique, une infection grave par le virus d'Epstein-Barr ou des cancers associés au virus d'Epstein-Barr, une leucémie lymphoblastique aiguë à cellules B, ou une insuffisance médullaire inexpliquée.

4. Mavorixafor ou un sel ou une composition pharmaceutiquement acceptable de celui-ci, pour utilisation selon l'une quelconque des revendications 1 à 3, dans lequel le mavorixafor ou un de ses sels pharmaceutiquement acceptables est administré à une dose d'environ 100 mg/jour à environ 600 mg/jour ; ou : dans lequel le mavorixafor ou un de ses sels pharmaceutiquement acceptables est administré à une dose d'environ 400 mg/jour.

5. Mavorixafor, ou un sel ou une composition pharmaceutiquement acceptable de celui-ci, pour utilisation dans un procédé visant à réduire le risque d'infection chez un patient atteint de neutropénie, dans lequel le patient atteint de neutropénie ne présente pas de mutation à gain de fonction dans le gène *CXCR4* et ne présente pas d'anomalie génétique associée au syndrome WHIM, ladite utilisation comprenant l'administration au patient d'une quantité efficace de mavorixafor ou d'un sel ou d'une composition pharmaceutiquement acceptable de celui-ci,
dans lequel du G-CSF est co-administré au patient, la dose de G-CSF administrée au patient étant réduite d'au moins 25 % par rapport à la dose précédente du patient avant le début du traitement par le mavorixafor ou un sel ou une composition pharmaceutiquement acceptable de celui-ci ; éventuellement :
dans lequel le patient présente une neutropénie chronique sévère (SCN), une neutropénie chronique idiopathique (CIN) ou une neutropénie auto-immune (AIN) ; et/ou
dans lequel le patient présente un nombre absolu de neutrophiles inférieur à 500 cellules/µL.

6. Mavorixafor, ou un sel ou une composition pharmaceutiquement acceptable de celui-ci, pour utilisation selon la revendication 5, dans lequel le patient présente des effets indésirables attribués au traitement par le G-CSF.

7. Mavorixafor, ou un sel ou une composition pharmaceutiquement acceptable de celui-ci, pour utilisation selon la revendication 5 ou la revendication 6, dans lequel le patient présente un risque accru d'infection choisie parmi les infections des voies respiratoires, l'otite moyenne, la stomatite, les infections des voies urinaires, la pyélonéphrite, les abcès cutanés, la cellulite et la septicémie.

8. Mavorixafor, ou un sel ou une composition pharmaceutiquement acceptable de celui-ci, pour utilisation selon l'une quelconque des revendications 5 à 7, dans lequel le mavorixafor ou un de ses sels pharmaceutiquement acceptables est administré à une dose d'environ 100 mg/jour à environ 600 mg/jour ; ou : dans lequel le mavorixafor ou un de ses sels pharmaceutiquement acceptables est administré à une dose d'environ 400 mg/jour.

9. Mavorixafor, ou un sel ou une composition pharmaceutiquement acceptable de celui-ci, pour utilisation dans un procédé visant à réduire la dose du facteur de stimulation des colonies de granulocytes (G-CSF) pour le traitement d'une neutropénie chronique sévère (SCN) chez un patient qui en a besoin, dans lequel le patient ne présente pas de mutation à gain de fonction dans le gène *CXCR4* et ne présente pas d'anomalie génétique associée au syndrome WHIM, ladite utilisation comprenant l'administration au patient d'une quantité efficace de mavorixafor ou d'un sel ou d'une composition pharmaceutiquement acceptable de celui-ci ; dans lequel la dose de G-CSF administrée au patient est réduite d'au moins environ 25 % par rapport à la dose précédente du patient avant le début du traitement par le mavorixafor ou un sel ou une composition pharmaceutiquement acceptable de celui-ci ;
éventuellement :
dans lequel la dose de G-CSF administrée au patient est réduite d'au moins environ 50 % par rapport à la dose précédente du patient avant le début du traitement par le mavorixafor ou un sel ou une composition pharmaceutiquement acceptable de celui-ci.

10. Mavorixafor, ou un sel ou une composition pharmaceutiquement acceptable de celui-ci, pour utilisation selon la revendication 9, dans lequel le mavorixafor ou un de ses sels pharmaceutiquement acceptables est administré à une dose d'environ 100 mg/jour à environ 600 mg/jour ; et/ou : dans lequel le mavorixafor ou un de ses sels pharmaceutiquement acceptables est administré à une dose d'environ 400 mg/jour.

11. Mavorixafor, ou un sel ou une composition pharmaceutiquement acceptable de celui-ci, pour utilisation selon la revendication 9 ou la revendication 10, dans lequel (I) le patient présente une neutropénie congénitale ; éventuellement dans lequel la neutropénie congénitale est choisie dans le groupe constitué de : la GSD1b due à des mutations dans le gène *SLC37A4* ; un déficit en G6PC3 dû à des mutations dans le gène *G6PC3*, et un déficit en GATA2 dû à des mutations dans le gène *GATA2* ; ou:
dans lequel (II) le patient présente une neutropénie chronique idiopathique (CIN).

12. Mavorixafor, ou un sel ou une composition pharmaceutiquement acceptable de celui-ci, pour utilisation selon l'une quelconque des revendications 9 à 11, dans lequel le patient présente un nombre absolu des neutrophiles (ANC) inférieur à 500 cellules/µL avant le traitement ; ou :
dans lequel le patient présente un nombre absolu des neutrophiles (ANC) inférieur à 600 cellules/µL avant le traitement.

13. Mavorixafor, ou un sel ou une composition pharmaceutiquement acceptable de celui-ci, pour utilisation selon l'une quelconque des revendications 9 à 12, dans lequel le patient présente des effets indésirables attribués à la thérapie avec le G-CSF; éventuellement dans lequel le procédé permet une diminution de l'incidence des douleurs osseuses chez le patient, une diminution de l'incidence des symptômes de type grippal chez le patient, ou une diminution de l'incidence d'une néoplasie myéloïde chez le patient.

14. Mavorixafor, ou un sel ou une composition pharmaceutiquement acceptable de celui-ci, pour utilisation selon l'une quelconque des revendications 1 à 13, dans lequel l'utilisation entraîne une augmentation du nombre absolu de neutrophiles (ANC) à au moins 800 cellules/µL ; ou les nombres de cellules des globules blancs (WBC), des neutrophiles et/ou des lymphocytes augmentent jusqu'à environ 2,0 fois la valeur de référence.
